# EUROPEAN PATENT SPECIFICATION

(11) **EP 2 270 747 B1**
(45) Date of publication and mention of the grant of the patent: **06.05.2015**
(21) Application number: 10167894.4
(22) Date of filing: 30.06.2010
(51) Int. Cl.: G06T 7/00, G01N 33/48, C12Q 1/68

(54) **Methods for detecting nucleic acid with microarray and program product for use in microarray data analysis**
Verfahren zur Detektion von Nukleinsäure mit Mikroarray und Programmprodukt zur Verwendung bei der Mikroarray-Daten-Analyse
Procédés pour détecter l'acide nucléique avec un microréseau et produit de programme à utiliser dans l'analyse de données à microréseau

(30) Priority: 30.06.2009 JP 2009155926; 24.03.2010 JP 2010068297
(43) Date of publication of application: 05.01.2011
(73) Proprietor: Sysmex Corporation, Kobe-shi, Hyogo 651-0073 (JP)
(72) Inventor: Ayako, Sakai, Kobe-shi, Hyogo 651-0073 (JP); Yoshihiko, Tashima, Kobe-shi, Hyogo 651-0073 (JP); Masahiro, Kajita, Kobe-shi, Hyogo 651-0073 (JP)
(74) Representative: Paemen, Liesbet R.J.

(56) References cited:
- US-A1- 2002 051 973
- US-A1- 2005 239 104
- RITCHIE MATTHEW E ET AL: "A comparison of background correction methods for two-colour microarrays", BIOINFORMATICS, OXFORD UNIVERSITY PRESS, SURREY, GB LNKD- DOI:10.1093/BIOINFORMATICS/BTM412, vol. 23, no. 20, 15 October 2007 (2007-10-15), pages 2700-2707, XP008128916, ISSN: 1367-4803
- SILVER JEREMY D ET AL: "Microarray background correction: maximum likelihood estimation for the normal-exponential convolution.", BIOSTATISTICS (OXFORD, ENGLAND) APR 2009 LNKD- PUBMED:19068485, vol. 10, no. 2, April 2009 (2009-04), pages 352-363, XP002609311, ISSN: 1468-4357

## Description

### TECHNICAL FIELD

The invention relates to methods of detecting a nucleic acid with a microarray or microarrays and a program product for use in microarray data analysis. More specifically, the invention relates to a microarray-based method of detecting a target nucleic acid containing a base sequence to which a nucleic acid-binding protein binds and to a microarray data analysis program product for use in the detection method.

### BACKGROUND ART

A microarray, which is also called "nucleic acid chip," includes a substrate such as a plastic or glass substrate and nucleic acid fragments that are placed as probes at high density on the substrate. Hybridization of the probes on the microarray to nucleic acids (DNA, cDNA, RNA, or cRNA) in an analyte sample allows quantitative or qualitative analysis of a number of genes contained in the nucleic acids in the analyte sample.

For example, using a microarray for RNA analysis makes it possible to perform gene expression analysis, and using a microarray for genomic DNA analysis makes it possible to measure the number of its copies (CGH analysis) and to analyze transcription factors and methylated regions of DNA.

Also known is a microarray-based method of detecting a target nucleic acid containing a base sequence to which a nucleic acid-binding protein binds. For example, the "ChIP-on-chip" method is known, which combines microarray analysis with chromatin immunoprecipitation (CHIP) method. In the ChIP-on-chip method, first, chromatin immunoprecipitation is performed using an antibody that recognizes a nucleic acid-binding protein of interest, so that a nucleic acid to which the protein binds is obtained. The nuclei acid obtained is then allowed to hybridize to probes on a microarray. The probes hybridizing to the nucleic acid on the microarray are then checked so that the nucleic acid to which the nucleic acid-binding protein of interest binds can be analyzed.

The "MeDIP-on-chip" method is also known, which combines microarray analysis with methylated DNA immunoprecipitation (MeDIP) method using an anti-methylated cytosine antibody or an anti-methylated cytidine antibody. In the MeDIP-on-chip method, methylated DNA can be analyzed by checking, on a microarray, probes hybridizing to DNA obtained by methylated DNA immunoprecipitation.

Exhaustive analysis of methylated DNA or nucleic acid to which nucleic acid-binding proteins bind is also performed using a tiling array in which probes having base sequences extracted at regular intervals from the whole genome region or a specific region are arranged like tiles.

In microarray analysis, the nucleic acid in the analyte sample usually hybridizes to probes having base sequences complementary to its base sequence. In some cases, however, the nucleic acid non-specifically hybridizes to the microarray substrate surface or probes having base sequences non-complementary to its base sequence. Signal measurement values obtained by such non-base-sequence-specific hybridization between nucleic acid and each probe may include biologically-groundless noise components (hereinafter referred to as "background"). Such a background is a significant cause of a reduction in measurement accuracy.

Therefore, microarray data analysis needs background correction, which is a process of correcting data by removal of a background caused by non-specific hybridization as described above.

For example, a known background correction method is performed using mismatch probes on GeneChip (registered trademark) from Affymetrix, Inc.

GeneChip (registered trademark) from Affymetrix, Inc., has perfect match (PM) probes, which have sequences completely complementary to the sequence to be analyzed, and mismatch (MM) probes having sequences different in only one base from the PM probe sequences. The signal values obtained from the MM probes are subtracted from the signal values obtained from the PM probes, so that the background caused by non-probe-sequence-specific binding is removed, which makes correction of the measurement data possible.

However, this method must use MM probes placed on the microarray and therefore is not applicable in cases where the microarray used has no MM probe. In many cases, non-specific hybridization has significantly different effects on PM and MM probes. Therefore, this method is not always considered to properly reflect the value free from the effect (see WO03/070938).

Background correction methods other than those using MM probes have also been developed (see WO03/070938). For example, a certain method for removing a background is performed using a region in which the measurement probes to be analyzed are not provided (see the BACKGROUND ART section of WO03/070938). This method may include obtaining a background signal from a region around each probe-carrying region (spot) or from a blank spot having no probe and subtracting the background signal from a signal obtained from the measurement probe. In this method, therefore, the measurement data can be corrected by removing the background caused by non-specific hybridization between the nucleic acid in the analyte sample and the microarray substrate surface.

In this method, however, the background caused by non-specific binding between the nucleic acid in the analyte sample and probes cannot be removed, which means that the accuracy of the data analysis is not so high.

In the blank spot-based method, a blank spot must be provided in advance on the substrate in addition to the probes having base sequences complementary to the base sequence to be analyzed. Therefore, this method is not applicable in cases where the microarray used has no blank spot.

There is another background correction method using random probes (see JP2008-039475). This method is performed using a microarray having a substrate provided with not only probes having base sequences complementary to the base sequence to be analyzed (counting probes) but also probes not corresponding to the base sequence to be analyzed (random probes). This method includes predicting the influence of the background based on the signal obtained from the random probes and removing the influence from the signal obtained from the counting probes.

Also in this method, however, random probes must be provided in advance on the substrate in addition to the counting probes. Therefore, this method is not applicable in cases where the microarray used has no blank spot. Also in this method, the influence of the non-specific binding may significantly vary with each random probe, depending on the analysis object, so that background correction sometimes cannot be performed enough.

Similarly, US2005/239104 makes use of a non-specific background control probe which is designed based on its dissimilarity to any known target that is expected to be in a test sample.

Besides the above background correction methods, there is a data correction method based on data normalization.

In microarray-based experiments, measured signal data often has a bias (deviation) varying from one measurement to another. Therefore, when different microarray data are compared, the bias between the microarrays has to be corrected by a mathematical or statistical method. Such a correction process is called normalization.

Data normalization may be used not only for correction of the bias between microarrays but also for correction of the bias caused by a positional difference between spots in a single microarray.

While an Affymetrix tiling array has no probe for background correction, Tiling Analysis Software (hereinafter referred to as "TAS"), which is an analysis program for data normalization, is offered by Affymetrix, Inc. In the analysis with TAS, signal data obtained from probes is not corrected by removal of a background but corrected by normalization using a mathematical or statistical method, and a significance probability (p value) between measurement data is calculated.

However, correction based on data normalization should be performed on the premise that the following two conditions are satisfied in the probes placed on a microarray. (1) Most of the gene probes do not show fluctuations in expression between measurements. (2) Gene probes that are contained in a microarray and show increase and decrease in expression between measurements are similar in number.

Therefore, such erroneous normalization that any of these conditions are not satisfied may rather cause a background. In correction based on data normalization, background correction is not performed, and therefore, the accuracy of the data analysis is not considered to be high.

### SUMMARY OF THE INVENTION

The scope of the present invention is defined solely by the appended claims, and is not affected to any degree by the statements within this summary.

An object of the invention is to provide a microarray-based method capable of detecting, with high accuracy from an analyte sample, a target nucleic acid containing a base sequence to which a nucleic acid-binding protein binds.

Another object of the invention is to provide a method in which background correction is possible even when special probes for background correction, such as MM probes or random probes as described above are not placed on the microarray in advance.

A further object of the invention is to provide a program product that enables a computer to perform analysis of microarray data obtained by the detection described above.

The inventors have found that in a microarray-based method of detecting, from an analyte sample, a target nucleic acid containing a base sequence to which a nucleic acid-binding protein binds (hereinafter also referred to as "recognition sequence"), a signal obtained from a correction probe that does not contain any sequence complementary to the recognition sequence may be used as a background to correct the signal obtained from a detection probe hybridizing to the target nucleic acid, so that the target nucleic acid can be detected from the analyte sample with high accuracy, and as a result, have made the invention.

The inventors have also found that when a correction probe is selected from probes placed on a microarray, based on a recognition sequence, a target nucleic acid can be detected with high accuracy from an analyte sample even when the microarray has no background correction probe provided in advance, and as a result, have completed the invention.

The invention also provides a microarray-based method of detecting a target nucleic acid containing a recognition sequence, which further includes the step of selecting the correction probe from the probes placed on the microarray, based on the recognition sequence, in the method described above.

The invention also provides a program product for microarray data analysis, which further includes instructions for the process of selecting the correction probe from the probes placed on the microarray based on the recognition sequence.

The application further also discloses micro array detection devices capable of detection of a target nucleic acid containing a base sequence to which a nucleic acid-binding protein binds with improved accuracy, which devices are configured to perform said detection making use on a correction probe selected from probes placed on a microarray, based on a recognition sequence suitable for use in the methods provided herein..

More particularly, the invention provides devices which are configured to perform the methods according to the invention described herein. According to particular embodiments, the micro array detection devices according to the invention make use of a computer program product as described herein.

According to the method of the invention, a target nucleic acid containing a recognition sequence can be detected with high accuracy from an analyte sample using a microarray. According to the invention, background correction is also possible even when any special probes for background correction are not placed on the microarray in advance. The program product of the invention enables a computer to perform microarray data analysis according to the detection method described above.

The methods and devices of the invention also make it possible to produce high-accuracy detection data from a microarray experiment in which a target nucleic acid containing a base sequence to which a nucleic acid-binding protein binds is detected from an analyte sample. Thus, the methods and devices of the invention can be used to find drug targets and to diagnose diseases.

### BRIEF DESCRIPTION OF THE DRAWINGS

Fig. 1 is a general schematic view of a microarray data analysis system including a microarray data analyzer to execute a program according to an embodiment of the invention;
Fig. 2 is a flow chart showing the process of microarray data analysis according to a first embodiment of the invention to be executed by a CPU 110a of an analyzer 100;
Fig. 3 is a flow chart showing the process of microarray data analysis according to a second embodiment of the invention to be executed by the CPU 110a of the analyzer 100;
Fig. 4 is a flow chart showing the process of microarray data analysis according to a third embodiment of the invention to be executed by the CPU 110a of the analyzer 100;
Fig. 5 is a flow chart showing the process of microarray data analysis according to a fourth embodiment of the invention to be executed by the CPU 110a of the analyzer 100;
Fig. 6 is a photograph of agarose gel electrophoresis showing that methylated DNA is specifically collected in Example 1;
Fig. 7 is a graph showing the results of microarray analysis (S/N ratios and significance probabilities) performed using a breast cancer cell line MCF-7 for the analyte sample;
Fig. 8 is a graph showing the results of microarray analysis (S/N ratios and significance probabilities) performed using a breast cancer cell line SK-BR-3 for the analyte sample;
Fig. 9 is a graph showing significance probabilities obtained in cases where background correction was performed using CpG sequence-free correction probes and using correction probes including control probes, respectively; and
Fig. 10 is a block diagram showing the hardware configuration of the analyzer 100 according to an embodiment of the invention.

### DETAILED DESCRIPTION OF THE PREFERRED EMBODIMENTS

The preferred embodiments of the present invention will be described hereinafter with reference to the drawings.

The term "nucleic acid-binding protein" means a protein that recognizes a specific base sequence of a nucleic acid and specifically binds to it. The nucleic acid-binding protein is preferably a DNA-binding protein. Examples of DNA-binding proteins include polycomb-group proteins, transcription factors, methylated DNA-binding proteins, anti-methylated cytosine antibodies, and anti-methylated cytidine antibodies.

The term "polycomb-group" means a molecular group of chromatin-binding large protein complexes present in cells. The term "transcription factor" means a gene transcription-regulating factor in a molecular group other than the RNA polymerase group. Examples of transcription factors include TATA-binding proteins (TBPs), EGR2, c-Myc, ER, and SOX6.

The term "base sequence to which a nucleic acid-biding protein binds" means a base sequence which the nucleic acid-binding protein is known to recognize and to which the nucleic acid-binding protein is known to specifically bind, and it is also called recognition sequence.

Such a recognition sequence preferably has a length of about 1 to about 15 bases, more preferably 1 to 10 bases, even more preferably 1 to 7 bases, most preferably 1 to 5 bases.

When the nucleic acid-binding protein is a methylated DNA-binding protein, the recognition sequence may be, for example, a CpG sequence. When the nucleic acid-binding protein is a transcription factor, the recognition sequence may be, for example, any one of the base sequences shown in Table 1.

Such a sequence to which a nucleic acid-binding protein binds can be found in databases known to those skilled in the art, such as TESS-General Factor Search Form (http://www.cbil.upenn.edu/cgi-bin/tess/tess?RQ=FCT-FRMREQ-Search).

**Table 1**

| |
|---|
| 1 Nucleic acid-binding protein |
| 2 TATA-binding protein |
| 3 Recognition sequence |

The term "CpG sequence" means a sequence in which cytosine (C) and guanine (G) are adjacent to each other in this order in the 5' to 3' direction. The letter "p" of CpG represents the phosphodiester bond between cytosine and guanine. In the description, "CpG sequence" and "CG sequence" are interchangeable. It is known that CpG sequences are modified by methylation in mammalian genomic DNAs.

The term "target nucleic acid containing a base sequence to which a nucleic acid-binding protein binds" (hereinafter also simply referred to as "target nucleic acid") means a nucleic acid that contains the recognition sequence in the base sequence and is a target for the detection in an embodiment of the invention. While the target nucleic acid may be any of DNA and RNA, it is preferably DNA, more preferably genomic DNA, even more preferably a genomic DNA containing a CpG sequence. In the detection of such a target nucleic acid, whether or not a genomic DNA in an analyte sample is methylated can be detected, and the methylated genomic DNA can also be detected.

The microarray that may be used in the method according to an embodiment of the invention is preferably a DNA microarray or a DNA chip, while it may be any microarray having nucleic acid probes placed on a substrate.

Such a microarray is also preferably a microarray having probes for detection and correction. However, some microarrays are not provided with special probes for background correction in advance so that a large number of probes for detection can be placed thereon. When such microarrays are used, a probe or probes for correction are selected from the probes present on the microarray, based on the base sequence to which the target nucleic acid-binding protein of interest binds, so that the method according to an embodiment of the invention can be performed.

The microarray may be a commercially-available product such as GeneChip (registered trademark) manufactured by Affymetrix, Inc., or a product prepared by methods known to those skilled in the art.

In an embodiment of the invention, the probe for detection (detection prove) may be any probe that hybridizes to the target nucleic acid. Specifically, a probe having a sequence complementary to a recognition sequence-containing region in the target nucleic acid may be used as the detection probe. More specifically, such a detection probe is a probe having a sequence complementary to the recognition sequence or a probe complementary to a region a region within 300 bases from the recognition sequence in the target nucleic acid.

The term "hybridize" means that the probe and the target nucleic acid form a double-stranded structure under stringent conditions. The stringent conditions may be any conditions that are usually used by those skilled in the art for the hybridization between a target nucleic acid and a probe or probes placed on a microarray.

In an embodiment of the invention, the probe for correction (correction probe) may be any probe that does not contain any sequence complementary to the recognition sequence. For example, when the nucleic acid-binding protein is an anti-methylated cytosine antibody or an anti-methylated cytidine antibody as described above, the correction probe is a CpG sequence-free probe. The correction probe is a probe that hybridizes to a nucleic acid other than the target nucleic acid (hereinafter also referred to as "non-target nucleic acid"). The non-target nucleic acid is preferably a nucleic acid whose base sequence is not only free of the probe-hybridizing region but also free of the recognition sequence in the vicinity of the probe-hybridizing region.

Therefore, the correction probe is a probe that does not contain any sequence complementary to the recognition sequence and hybridizes to a non-target nucleic acid that does not contain the recognition sequence in a region within 300 bases from the region to which the probe hybridizes.

A single detection probe and a single correction probe may be provided on the microarray used in the method and/or device according to an embodiment of the invention. Generally, however, in order to achieve higher detection accuracy, two or more detection probes and two or more correction probes are preferably provided.

In the first step of the method according to an embodiment of the invention, an analyte sample is brought into contact with a microarray having detection and correction probes. The contact may be achieved by adding the analyte sample to the microarray.

The contact between the analyte sample and the microarray may generally be performed at an ambient temperature (about 10 to 70°C) for 2 to 20 hours, depending on the type of the microarray.

The analyte sample potentially contains the target nucleic acid. The analyte sample may be a nucleic acid-containing sample obtained from a biological sample. Examples of biological samples include cultured cell strains, blood, serum, lymph fluid, urine, papillary secretions, body fluids, and tissues or cells collected by surgery or biopsy.

The analyte sample may be any of these biological samples themselves or a product obtained by subjecting the biological sample to a treatment for increasing the concentration of the target nucleic acid in the analyte sample. Examples of treatments for increasing the target nucleic acid concentration include immunoprecipitation methods using an antibody to the target nucleic acid or an antibody to the target nucleic acid-biding protein, and nucleic acid amplification methods using primers to the target nucleic acid. The analyte sample is preferably obtained by performing both immunoprecipitation and nucleic acid amplification.

The target nucleic acid contained in the analyte sample may contain one or more recognition sequences.

The immunoprecipitation may be performed by any known method capable of precipitating the target nucleic acid with an antibody to the nucleic acid. When the target nucleic acid is a genomic DNA containing a methylated CpG sequence, a MeDIP method with an anti-methylated cytosine antibody or an anti-methylated cytidine antibody may be used as the immunoprecipitation method. When the target nucleic acid contains a base sequence to which a transcription factor binds, CHIP methods with various anti-transcription factor antibodies may be used as the immunoprecipitation method.

The nucleic acid amplification method may be any method capable of amplifying the target nucleic acid with primers to the nucleic acid, and amplification methods known to those skilled in the art may be performed, such as polymerase chain reaction (PCR) methods, in vitro transcription (IVT) amplification methods, and SPIA (trademark) amplification process. The primers to be used in the nucleic acid amplification can be properly designed by those skilled in the art according to the base sequence of the target nucleic acid, or commercially available primers may be used.

The nucleic acid may be fragmented using an ultrasonic wave or a restriction enzyme before or after the immunoprecipitation method and/or the nucleic acid amplification method.

The target nucleic acid contained in the analyte sample is preferably labeled with a marker known in the art. Therefore, the method according to an embodiment of the invention preferably further includes the step of labeling the target nucleic acid. The labeling step is advantageously performed after the step of amplifying the target nucleic acid, because all the molecules of the target nucleic acid in the analyte sample can be labeled in the labeling step.

Examples of the marker include fluorescent materials, haptens such as biotin, radioactive substances, and so on. Examples of fluorescent materials include Cy3, Cy5, Alexa Fluor (trademark), FITC, and so on. When the nucleic acid to be detected is labeled as described above, the signal is easily measured in the next step.

Methods for labeling the nucleic acid with the marker are known in the art.

In the second step of the method according to an embodiment of the invention, first and second signals obtained from the detection and correction probes, respectively, are measured, so that first and second signal measurement values are obtained, respectively.

The first and second signal measurement values may each be a single value. Since the microarray generally has a plurality of detection probes and a plurality of correction probes, a plurality of measurement values may be obtained for each signal.

The signals may be signals suitable for the type of the microarray. For example, the signals may be electric signals which are generated in the presence of a nucleic acid hybridizing to each probe of the microarray, or when the nucleic acid to be detected is labeled as described above, the signals may be fluorescence signals, luminescence signals or the like generated from the marker. These signals are preferably signals generated from the marker, more preferably fluorescence signals generated from the marker.

Each of the signals may be detected using a scanner installed in a general microarray analyzer. For example, the scanner may be GeneChip (registered trademark) Scanner 3000 7G (Affymetrix, Inc.).

The second signal obtained from the correction probe should be 0 (not measured). In general, however, the second signal may be measured to be a certain value due to non-specific hybridization between a nucleic acid and the probe and/or the substrate of the microarray.

In the third step according to an embodiment of the invention, a background value is obtained based on the second signal measurement value. When the microarray has a plurality of correction probes, the background value may be a statistically representative value obtained from a plurality of second signal measurement values. While the statistically representative value may be any one of a maximum value, a minimum value, a mean value, a median value, or a mode value, it is preferably a mode value.

In the fourth step of the method according to an embodiment of the invention, the first signal measurement value is corrected using the background value, so that a first signal correction value is obtained.

When the microarray has a plurality of detection probes, each first signal measurement value is corrected using the background value, so that each first signal correction value is obtained.

The correction may be made by dividing the first signal measurement value by the background value or by subtracting the background value from the first signal measurement value. Preferably, the correction value is calculated by subtracting the background value from the first signal measurement value. If the correction value obtained by the subtraction is negative, the correction value may be assumed to be 0.

In the fifth step of the method according to an embodiment of the invention, the target nucleic acid contained in the analyte sample is detected based on the first signal correction value.

As used herein, the term "detect" means the detection of whether or not the target nucleic acid is present in the analyte sample and/or the determination of the amount of the target nucleic acid present in the analyte sample, preferably the detection of whether or not the target nucleic acid is present.

In this step, the first signal correction value may be compared with a predetermined threshold value. If the first signal correction value is higher than the threshold value, the target nucleic acid may be determined to be present in the analyte sample. If it is lower than the threshold value, the target nucleic acid may be determined to be absent from the analyte sample.

The predetermined threshold value may be empirically derived from accumulated correction value data, which are measured by the same procedure as in the method according to an embodiment of the invention, using samples which have previously been confirmed for the presence or absence of the nucleic acid to be detected. For example, a correction value that is obtained from samples which have previously been confirmed for the presence or absence of the nucleic acid to be detected may be used as the threshold value. The mean, median or mode value of correction values obtained from two or more samples may also be used as the threshold value.

The method according to an embodiment of the invention may include adding an analyte sample and a control sample to a plurality of microarrays, respectively, obtaining a correction value from each microarray by the method described above, and obtaining an analysis value based on the correction values so that the target nucleic acid can be detected.

In such a method according to an embodiment of the invention, the same two microarrays are preferably provided, one of which is preferably used as a microarray A, and the other of which is preferably used as a microarray B.

When two microarrays are used as described above, the method according to an embodiment of the invention includes the steps of:
(1) bringing an analyte sample potentially containing a recognition sequence-containing target nucleic acid into contact with a microarray A having a detection probe capable of hybridizing to the target nucleic acid and a correction probe not containing any sequence complementary to the recognition sequence and bringing a control sample with no possibility of containing the target nucleic acid into contact with a microarray B having the same probes as the microarray A;
(2) after the contact step, measuring a first analyte signal obtained from the detection probe of the microarray A and a second analyte signal obtained from the correction probe of the microarray A, so that a first analyte signal measurement value and a second analyte signal measurement value are obtained, respectively, and measuring a first control signal obtained from the detection probe of the microarray B and a second control signal obtained from the correction probe of the microarray B, so that a first control signal measurement value and a second control signal measurement value are obtained, respectively;
(3) obtaining an analyte background value and a control background value, respectively, based on the second analyte signal measurement value and the second control signal measurement value;
(4) correcting the first analyte signal measurement value with the analyte background value to obtain an analyte correction value for the first analyte signal, and correcting the first control signal measurement value with the control background value to obtain a control correction value for the first control signal;
(5) obtaining an analysis value based on the analyte correction value and the control correction value; and
(6) detecting the target nucleic acid contained in the analyte sample, based on the analysis value.

In the step (1), the analyte sample is brought into contact with the microarray A, and the control sample is brought into contact with the microarray B.

As described above, the analyte sample potentially contains the target nucleic acid. On the other hand, the control sample has no possibility of containing the target nucleic acid. For example, the control sample may be obtained by subjecting the biological sample to immunoprecipitation using an antibody that does not specifically recognize the target nucleic acid or the target nucleic acid-binding protein (such as a normal mouse IgG antibody).

The contact may be performed in the same manner as described above for the first step.

In the step (2), the first analyte, second analyte, first control, and second control signal measurement values may each be a single value. In general, however, the microarrays A and B each have a plurality of detection probes and a plurality of correction probes, and therefore, a plurality of measurement values may be obtained for each signal.

The signal and the detection of the signal may be the same as those described above for the second step.

In the step (3), when the microarrays A and B each have a plurality of correction probes, the analyte background value and the control background value may be the maximum, minimum, mean, median, or mode of the second analyte signal measurement values and the second control signal measurement values, respectively, and they are each preferably the mode.

In the step (4), when the microarrays A and B each have a plurality of detection probes, analyte correction values are obtained by subtracting the analyte background value from first analyte signal measurement values, and control correction values are obtained by subtracting the control background value from first control signal measurement values. If each resulting correction value is negative, the correction value may be assumed to be 0.

In the step (5), the analysis value may be obtained as a significance probability. Specifically, the analysis value may be calculated by the Wilcoxon signed-rank test using the analyte correction value and the control correction value as a pair of samples. Alternatively, the analysis value may be a signal/noise (S/N) ratio calculated by dividing the analyte correction value by the control correction value.

As used herein, the term "significance probability", which is also called "p-value," has the same meaning as used generally in the art and is intended to include the probability of observing a statistic more extreme than the one actually calculated from the data obtained assuming that the null hypothesis is true (a statistic being contrary to the null hypothesis).

In the step (6), the analysis value is compared with a predetermined threshold value, and when the analysis value is lower than the predetermined threshold value, it may be determined that the target nucleic acid is detected from the analyte sample due to specific hybridization between the target nucleic acid and the detection probe. When the analysis value is the significance probability, the predetermined threshold value may be a significance level used generally in the art. For example, when the resulting analysis value is lower than a threshold value of 0.05 (5%), it is indicated that the probability that a non-target nucleic acid is erroneously detected by non-specific hybridization to the detection probe is 5% or less.

The threshold value used as a significance level is generally 0.05 (5%), preferably 0.01 (1%), more preferably 0.008 (0.8%).

In the method according to an embodiment of the invention using a plurality of microarrays, microarrays A and B having no correction probe placed in advance may be used. In this case, correction probes may be selected from the probes placed on these microarrays based on the recognition sequence so that the method described above can be performed. Specifically, probes that do not contain any sequence complementary to the recognition sequence may be selected for correction from the probes placed on the microarrays.

The correction probes are preferably selected based on the information about the genomic DNA base sequence of the organism from which the the nucleic acid contained in the analyte sample is derived. This is because any nucleic acid in the analyte sample, to which the correction probe can hybridize, should also preferably be free of the recognition sequence. In particular, therefore, a probe that does not contain any sequence complementary to the recognition sequence and hybridizes to a genomic DNA that does not contain the recognition sequence in the region within 300 bases from the probe-hybridizing region is preferably selected as a correction probe.

In the method according to an embodiment of the invention, detection probes may also be selected from the probes placed on the microarrays. In this case, the detection probes may be selected based on the base sequences of the probes placed on the microarray, the recognition sequence, and the base sequence of the genomic DNA of the organism from which the nucleic acid contained in the analyte sample is derived. Specifically, a probe having a sequence complementary to the recognition sequence or a probe complementary to a region within 300 bases from the recognition sequence in the genomic DNA is selected as a detection probe.

The method according to an embodiment of the invention may further include the step of performing correction by data normalization.

In the method according to an embodiment of the invention, the methylated state of a genomic DNA may be analyzed using an anti-methylated cytosine antibody or an anti-methylated cytidine antibody as the DNA-binding protein and using a CpG sequence-free probe as a correction probe.

The term "methylated state" means whether a cytosine residue of a CpG sequence present in the analyzed genomic DNA base sequence is methylated or not or the ratio of the number of the methylated CpG sequences to the number of all the CpG sites present in the base sequence.

A description is given below of the program for microarray data analysis according to an embodiment of the invention.

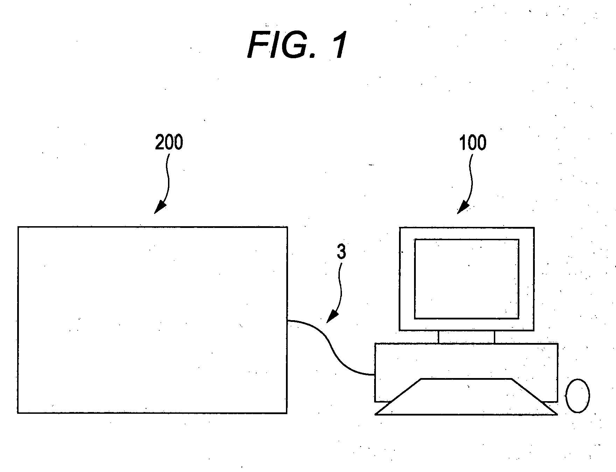

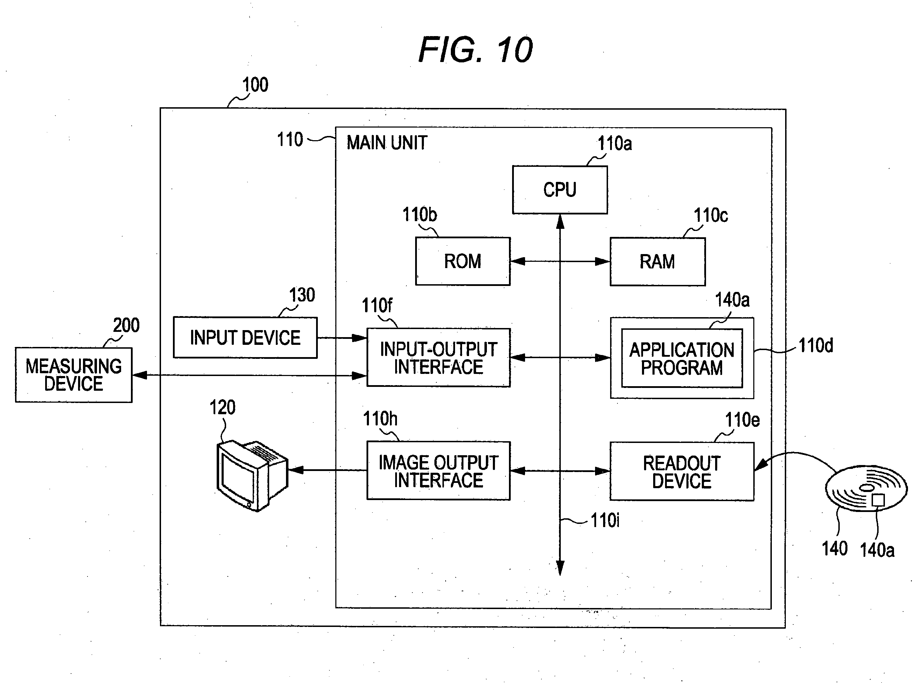

Fig. 1 shows a general schematic diagram of a microarray data analysis system including a microarray data analyzer 100 for implementing the program according to an embodiment of the invention. The microarray data analysis system includes a microarray measuring device 200 and the analyzer 100, which are connected to each other through a cable 3. Data such as a signal measurement value obtained from the microarray measured by the measuring device 200 is sent to the analyzer 100 through the cable 3. It will be understood that the measuring device 200 and the analyzer 100 may be configured into an integrated system. Fig. 10 is a block diagram showing the hardware configuration of the analyzer 100.

The analyzer 100 mainly includes a main unit 110, a display unit 120 and an input device 130. The main unit 110 includes a CPU 110a, a ROM 110b, a RAM 110c, a hard disk 110d, a readout device 110e, an input-output interface 110f, and an image output interface 110h, which are connected to one another through a bus 110i to allow data communication.

The CPU 110a can execute the computer program stored in the ROM 110b and the computer program loaded on the RAM 110c.

The ROM 110b includes a mask ROM, PROM, EPROM, EEPROM, or the like. The ROM 110b stores the computer program to be executed by the CPU 110a and the data to be used for the execution.

The RAM 110c includes an SRAM, DRAM or the like. The RAM 110c is used to read out the computer program stored in the RAM 110c, ROM 110b and hard disk 110d. When these computer programs are executed, the RAM 110c is also used as a work area for the CPU 110a.

Accordingly, the invention also provides micro array detection devices capable of, with improved accuracy. Typically, the devices according to the invention comprise a micro-array measuring device 200 and an analyzer 100, as described above. More particularly, the devices according to the invention are configured to:
obtain a first signal measurement value derived from a detection probe that hybridizes to the target nucleic acid and is placed on a microarray in contact with an analyte sample potentially containing the target nucleic acid containing a base sequence to which a nucleic acid-binding protein binds, and
a second signal measurement value derived from a correction probe that is placed on the microarray and does not contain any sequence complementary to the base sequence to which the nucleic acid-binding protein binds;
obtain a background value based on the second signal measurement value;
obtain a first signal correction value by correcting the first signal measurement value with the background value; and
to output the first signal correction value.

The invention more particularly provides micro array detection devices which are configured to detect a target nucleic acid containing a base sequence to which a nucleic acid-binding protein binds according to the methods described herein. More particularly, the micro array detection devices according to the invention make use of a computer program product as described herein. In further particular embodiments, the micro array detection devices according to the invention comprise a micro array A and B as described herein.

Various computer programs such as an operating system and an application system program to be executed by the CPU 110a and the data to be used for the execution of the computer programs are installed on the hard disk 110d. An application program 140a as described below is also installed on the hard disk 110d. Examples of the data to be used for the execution of the application program 140a include the recognition sequence, the base sequences of the microarray, and the base sequence of a genomic DNA, and so on.

The readout device 110e includes a flexible disk drive, a CD-ROM drive, or a DVD-ROM drive or the like. The readout device 110e can read out the computer program or data stored on a transportable storage medium 140. An application program 140a to allow the computer to execute the operation is also stored on the transportable storage medium 140. The CPU 110a may read out the application program 140a from the transportable storage medium 140, and the application program 140a may be installed on the hard disk 110d.

An operating system to provide a graphical user interface environment, such as Windows (registered trademark) manufactured and sold by Microsoft Corporation in the United States is installed on the hard disk 110d. A description will be given below, provided that the application program 140a for the decision described above runs on the operating system.

For example, the input-output interface 110f includes a serial interface such as USB, IEEE 1394 or RS-232C, a parallel interface such as SCSI, IDE or IEEE 1284, and an analog interface including a D/A converter, an A/D converter or the like. The input device 130 including a keyboard and a mouse is connected to the input-output interface 110f. The user can input data to the computer main unit 110 using the input device 130.

The microarray measuring device 200 is also connected to the input-output interface 110f through the cable 3. Therefore, the computer main unit 110 can receive the signal measurement values, which are obtained from probes placed on the microarray, from the microarray measuring device 200 through the input-output interface 110f.

The image output interface 110h is connected to the display unit 120 including an LCD, CRT or the like so that an image signal corresponding to the image data sent from the CPU 110a can be output on the display unit 120. The display unit 120 outputs the image data according to the image signal input. The display unit 120 also outputs the correction value or the analysis value sent from the CPU 110a as described below.

The application program 140a according to an embodiment of the invention is a program to allow the CPU 110a of the analyzer 100 to execute microarray data analysis.

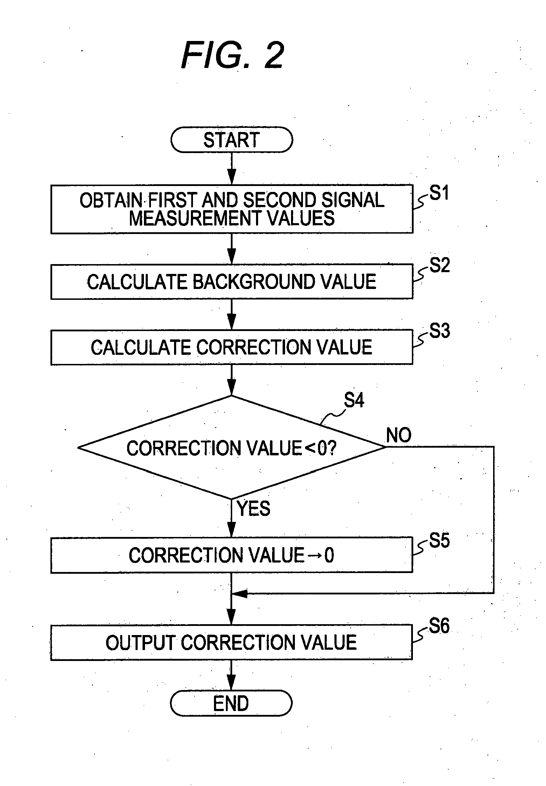

Fig. 2 is a flow chart showing the process of microarray data analysis according to a first embodiment of the invention to be executed by the CPU 110a of the analyzer 100.

A microarray including detection and correction probes each in contact with an analyte sample to be measured is placed in the microarray measuring device 200, and a first signal generated from the detection probe and a second signal generated from the correction probe are measured.

The CPU 110a of the analyzer 100 receives a first signal measurement value and a second signal measurement value from the microarray measuring device 200 through the input-output interface 110f (Step S1), and stores the first and second signal measurement values on the hard disk 110d.

The CPU 110a reads out the second signal measurement value from the hard disk 110d and calculates the mode of the second signal measurement value as a statistically representative value (Step S2), which is stored as a background value on the hard disk 110d of the analyzer 100. When the microarray has a plurality of correction probes, the statistically representative value is calculated from a plurality of second signal measurement values.

The CPU 110a reads out the first signal measurement value and the background value from the hard disk 110d and calculates a correction value by subtracting the background value from the first signal measurement value (Step S3). When the microarray has a plurality of detection probes, correction values are calculated by subtracting the background value from a plurality of first signal measurement values.

The CPU 110a determines whether or not the calculated correction value is negative (Step S4).

If the calculated correction value is determined to be negative in Step S4, the CPU 110a assumes the correction value to be "0" (Step S5). If the calculated correction value is determined not to be negative in Step S4, the calculated correction value is used as it is.

The CPU 110a outputs the correction value resulting from the calculation on the display unit 120 such as a display (Step S6).

In the first embodiment of the invention, the CPU 110a calculates the mode as a non-limiting statistically representative value. The statistically representative value may be the maximum, minimum, mean, median, or mode value. The mode value is preferred.

In the first embodiment, the correction value is calculated by subtracting the background value from the first signal measurement value. However, the correction is not limited to that, and the correction value may be calculated by dividing the first signal measurement value by the background value. Preferably, the first signal correction value is calculated by subtracting the background value from the first signal measurement value.

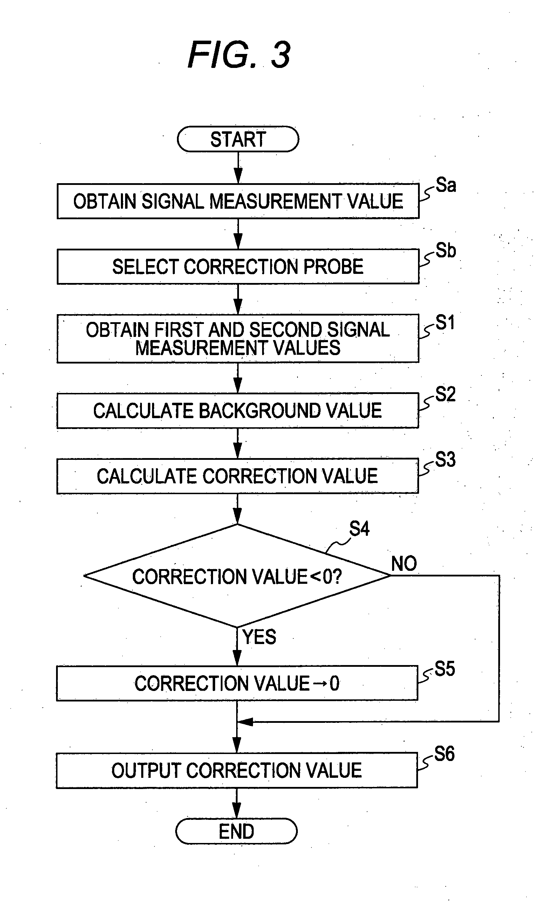

Fig. 3 is a flow chart showing the process of microarray data analysis according to a second embodiment of the invention to be executed by the CPU 110a of the analyzer 100 in a case where the microarray used has no special probe identified in advance for background correction. In this embodiment, the data on the recognition sequence and the base sequences of the probes placed on the microarray has been previously stored on the hard disk 110d.

The microarray in contact with an analyte sample to be measured is placed in the microarray measuring device 200 so that signals generated from probes can be measured.

The CPU 110a of the analyzer 100 receives signal measurement values from the microarray measuring device 200 through the input-output interface 110f (Step Sa), and stores them on the hard disk 110d of the analyzer 100.

The CPU 110a reads out the recognition sequence and the base sequences of the probes of the microarray from the hard disk 110d, selects, as a correction probe(s), a probe(s) that does not contain any base sequence complementary to the recognition sequence (hereinafter also referred to as "complementary sequence") (Step Sb), and stores the selected probe(s) on the hard disk 110d of the analyzer 100. The correction probe(s) is selected from the probes of the microarray by this process.

The CPU 110a reads out signal measurement values from the hard disk 110d to obtain a first signal measurement value derived from the detection probe and a second signal measurement value derived from the correction probe (Step S1), and stores these values on the hard disk 110d of the analyzer 100.

After Step S1, Steps S2 to S6 are performed in the same manner as described above in the processing procedure performed by the CPU 110a.

In the second embodiment, the data on the recognition sequence and the base sequences of the probes placed on the microarray has been previously stored on the hard disk 110d, which is not limiting. For example, the CPU 110a may receive the data on the recognition sequence input from the input device, through the input-output interface 110f. Alternatively, the data on the base sequences of the probes placed on the microarray may be stored in an external memory, and the CPU 110a may receive the data through the input-output interface 110f connected to the Internet. Alternatively, the data on the recognition sequence and the base sequences of the probes placed on the microarray may be recorded on a transportable storage medium 140, and the CPU 110a may receive the data read out from the readout device.

The base sequence of a genomic DNA of the organism from which the nucleic acid contained in the analyte sample is derived may also be stored on the hard disk 110d. In this case, Step Sb includes selecting a correction probe from the probes of the microarray based on the recognition sequence and the base sequence of the genomic DNA.

As described above, any nucleic acid in the analyte sample, to which the correction probe can hybridize, also preferably does not contain the recognition sequence. When the information about the base sequence of the genomic DNA is used, therefore, a probe capable of hybridizing to the genomic DNA that does not containing the recognition sequence in the region within 300 bases of the probe hybridizing region can be selected as the correction probe.

In a third embodiment of the invention, the CPU 110a of the analyzer 100 may make the selection of a detection probe(s) as well as a correction probe(s). In this case, the Step Sb includes allowing the CPU 110a to further read out the base sequence of the genomic DNA from the hard disk 110d and selecting a detection probe(s) from the probes of the microarray, based on the recognition sequence and the base sequence of the genomic DNA.

In this embodiment, the application program 140a may also allow the CPU 110a of the analyzer 100 to perform a method of detecting the target nucleic acid by adding an analyte sample and a control sample to a plurality of microarrays, respectively.

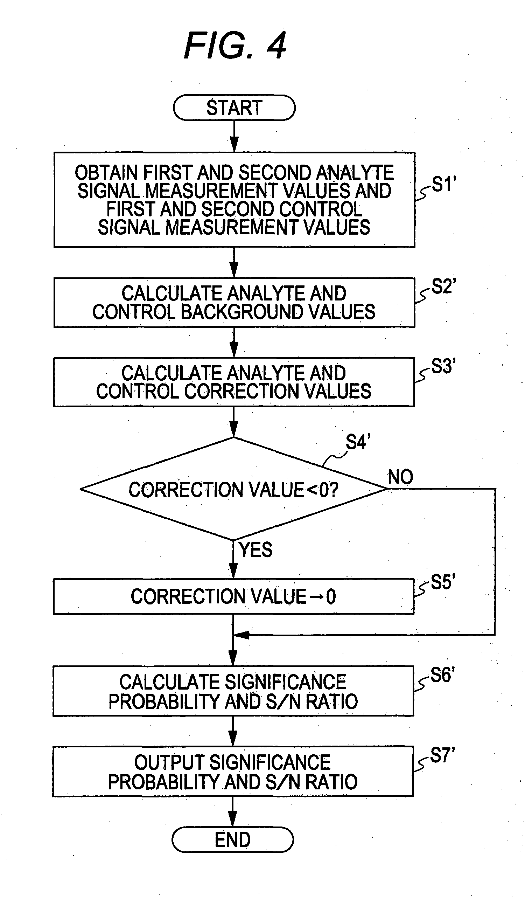

Fig. 4 is a flow chart showing the process of microarray data analysis according to the third embodiment to be executed by the CPU 110a of the analyzer 100 in a case where the same two microarrays (microarrays A and B) are used.

The microarray A having detection and correction probes each in contact with an analyte sample is placed in the microarray measuring device 200, and a first analyte signal generated from the detection probe and a second analyte signal generated from the correction probe are measured. In a similar manner, the microarray B having detection and correction probes each in contact with a control sample is placed in the microarray measuring device 200, and a first control signal generated from the detection probe and a second control signal generated from the correction probe are measured.

The CPU 110a of the analyzer 100 receives a first analyte signal measurement value, a second analyte signal measurement value, a first control signal measurement value, and a second control signal measurement value from the microarray measuring device 200 through the input-output interface 110f (Step S1') and stores these signal measurement values on the hard disk 110d in the computer.

The CPU 110a reads out the second analyte signal measurement value and the second control signal measurement value from the hard disk 110d, calculates the modes of the signal measurement values as statistically representative values (Step S2'), and stores these values as an analyte background value and a control background value on the hard disk 110d of the analyzer 100. When the microarrays A and B each have a plurality of correction probes, the mode of a plurality of second analyte signal measurement values and the mode of a plurality of second control signal measurement values are each calculated as a statistically representative value.

The CPU 110a reads out the first analyte signal measurement value and the analyte background value from the hard disk 110d and calculates an analyte correction value by subtracting the analyte background value from the first analyte signal measurement value. The CPU 110a also calculates a control correction value in a similar manner (Step S3'). When the microarrays A and B each have a plurality of detection probes, correction values are calculated by subtracting the analyte background value and the control background value from a plurality of first analyte signal measurement values and a plurality of first control signal measurement values, respectively.

The CPU 110a determines whether or not the calculated correction value is negative (Step S4').

If the calculated correction value is determined to be negative in Step S4', the CPU 110a assumes the correction value to be "0" (Step S5'a). If the calculated correction value is determined not to be negative in Step S4', the calculated correction value is used as it is.

The CPU 110a calculates a significance probability from the analyte correction values and the control correction values by the Wilcoxon signed-rank test and/or an S/N ratio by dividing the analyte correction value by the control correction value as an analysis value (Step S6'). These values are obtained in the same way as described above for the step (5) of the method according to an embodiment of the invention using two microarrays.

The CPU 110a outputs the calculated analysis value from the display unit 120 such as a display (Step S7').

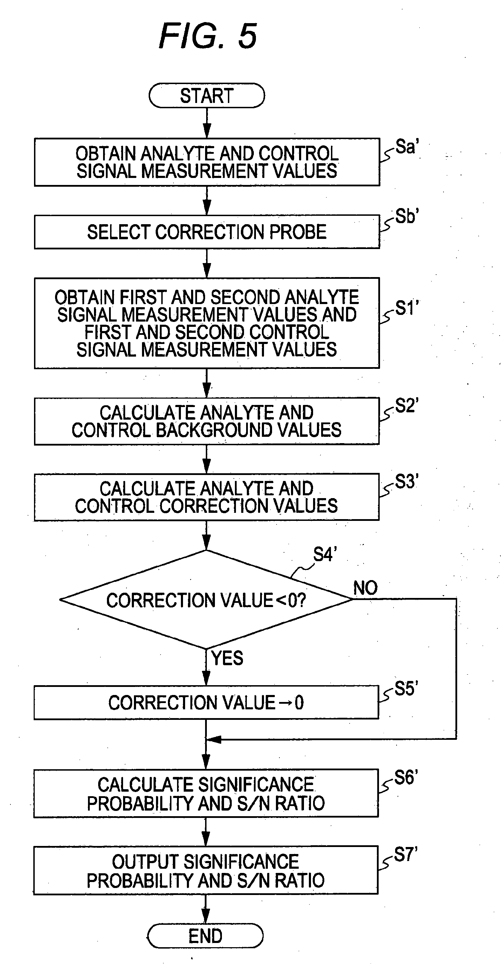

Fig. 5 is a flow chart showing the process of microarray data analysis according to a fourth embodiment of the invention to be executed by the CPU 110a of the analyzer 100 in a case where microarrays A and B used have no special probe identified in advance for background correction. In this embodiment, the data on the recognition sequence and the base sequences of the probes placed on the microarrays has been previously stored on the hard disk 110d.

The microarray in contact with an analyte sample is placed in the microarray measuring device 200, and signals generated from the probes are measured.

The microarray A in contact with an analyte sample is placed in the microarray measuring device 200, and analyte signals generated from the probes are measured. The microarray B in contact with a control sample is also placed in the microarray measuring device 200, and control signals generated from the probes are measured.

The CPU 110a of the analyzer 100 receives analyte signal measurement values and control signal measurement values from the microarray measuring device 200 through the input-output interface 110f (Step Sa'), and stores these values on the hard disk 110d of the analyzer 100.

The CPU 110a reads out the recognition sequence and the base sequences of the probes of the microarrays from the hard disk 110d, selects complementary sequence-free probes as correction probes (Step Sb'), and stores the selected probes on the hard disk 110d of the analyzer 100. In this process, the correction probes are selected from the probes of the microarrays.

The CPU 110a reads out analyte signal measurement values and control signal measurement values from the hard disk 110d, obtains first analyte and control signal measurement values derived from the detection probes and second analyte and control signal measurement values derived from the selected correction probes (Step S1'), and stores these values on the hard disk 110d of the analyzer 100.

After Step S1', Steps S2' to S7' are performed in the same manner as described above in the processing procedure performed by the CPU 110a.

In the fourth embodiment, the CPU 110a of the analyzer 100 may also make the selection of detection probes as well as the correction probes. In this case, the Step Sb' includes allowing the CPU 110a to further read out the base sequence of the genomic DNA from the hard disk 110d and to select detection probes from the probes of the microarrays, based on the recognition sequence and the base sequence of the genomic DNA.

The invention is more specifically described by the examples below, which are not intended to limit the scope of the invention.

### EXAMPLES

### Example 1

A target nucleic acid containing a base sequence to which a nucleic acid-binding protein binds was detected using an analysis method according to the invention and a conventional analysis method with Affymetrix TAS, respectively. In this example, the nucleic acid-binding protein is an anti-methylated cytosine antibody, and the base sequence to which the nucleic acid-binding protein binds is a CpG sequence.

In this example, specific procedures were performed according to the instructions in the manuals attached to the kit and reagents used.

### Preparation of Analyte Sample

### (1) Methylated DNA Immunoprecipitation Method

A genomic DNA (4 µg) was extracted from each of breast cancer-derived cell lines MCF-7, MB-MDA231 and SK-BR-3, and a mammary gland epithelial cell line HMEC and used as a biological sample. Each biological sample was allowed to react with a restriction enzyme Msel (New England Biolabs, Inc.) at 37°C overnight to form fragments of 300 to 1,000 bp. After the reaction, each biological sample was denatured by heating at 95°C for 10 minutes, so that a single-stranded genomic DNA was obtained.

The denatured biological sample was diluted with the dilution buffer attached to Chromatin Immunoprecipitation Assay Kit (Upstate Biotechnology Inc.). Protein G Sepharose beads (GE Healthcare) were then added to each resulting dilution. The mixture was rotated at 4°C for 30 minutes and then centrifuged, so that proteins non-specifically binding to the beads were removed. After the centrifugation, each supernatant was collected and divided into two aliquots, which were transferred to separate tubes. An anti-methylated cytosine antibody (for an analyte sample) was added to one of the tubes, and a normal mouse IgG antibody (Santa Cruz Biotechnology, Inc (for a control sample)) was added to the other tube. They were rotated at 4°C overnight.

After the overnight rotation, Protein G Sepharose beads (GE Healthcare) were then added and rotated at 4°C for 1 hour, so that the complex of the genomic DNA and the antibody binding thereto was allowed to bind to the beads. Thereafter, the mixtures were centrifuged. The supernatant was removed, and the beads were collected. After the collected beads were washed with the cleaning buffer attached to the assay kit, the genomic DNA was eluted from the immuneprecipitated complex using an elution buffer.

The genomic DNA obtained by the methylated DNA immunoprecipitation method was allowed to react with proteinase K and then purified with Qiaquick RCR Purification Kit (QIAGEN), so that an analyte sample and a control sample were obtained.

### (2) Check of Analyte Sample

PCR and agarose electrophoresis were performed to check whether the methylated DNA was specifically collected by the methylated DNA immunoprecipitation method (1).

### (i) Preparation of RCR Reaction Solution

The reagents shown below were mixed to form a 25 µl reaction solution.
2x fastStart SYBR Green Master Mix (Roche) 12.5 µl
Forward (F)-primer (10 µM) 1 µl
Reverse (R)-primer (10 µM) 1 µl
Genomic DNA (0.4 ng/µl) 1 µl
dH₂O 9.5 µl

The sequences of the primers used are as follows.
GSTP1 primer
F: GAGGCCTTCGCTGGAGTT (SEQ ID NO: 1)
R: GTACTCACTGGTGGCGAAGA (SEQ ID NO: 2)
ER primer
F: GCCTACGAGTTCAACGCCG (SEQ ID NO: 3)
R: AACGCCGCAGCCTCAGAC (SEQ ID NO: 4)
ch14-cgf1 primer
F: GGAGGAGTCAAGAGAAGTTGGAAGC (SEQ ID NO: 5)
R: CCCACACTCCATTTCCATTCCTC (SEQ ID NO: 6)

### (ii) PCR Reaction Conditions

PCR was performed using the above reaction solution under the following conditions: 95°C for 10 minutes; 45 cycles of 95°C for 30 seconds, 66°C for 15 seconds and 72°C for 30 seconds; and 1 cycle of 95°C for 1 minute, 66°C for 30 seconds and 95°C for 30 seconds.

### (iii) Agarose Electrophoresis

Each of the PCR products was subjected to 2% agarose gel electrophoresis for checking the amplified nucleic acid.

The GSTP1 gene is known to have a promoter region which is modified by methylation in MCF-7 cells but not modified by methylation in MB-MDA231 and HMEC cells. The ER gene is known to have a promoter region which is modified by methylation in SK-BR-3 cells but not modified by methylation in MCF-7 cells. The ch14-cgf1 gene is known not to be modified by methylation.

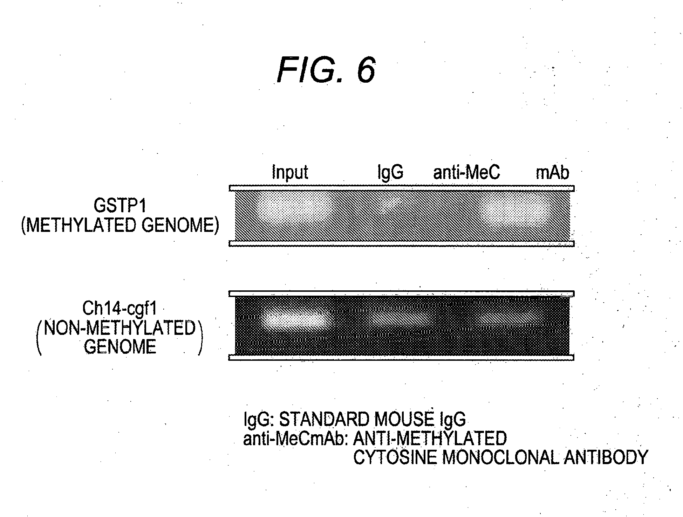

Fig. 6 shows a photograph of the gels in which the PCR amplification products were subjected to the agarose electrophoresis.

The results in Fig. 6 showed that the methylated DNA in the promoter region of the CSTP1 gene of the MCF-7 cell was specifically collected by the immunoprecipitation method using an anti-methylated cytosine antibody.

### (3) Amplification and Labeling of Nucleic Acid in Analyte Sample

The nucleic acid contained in the analyte sample and the control sample was amplified using WT-Ovation (trademark) Pico RNA Amplification System Version 1.0 (NuGEN). The concentration of the amplified nucleic acid was determined by measuring the absorbance (at 260 nm and 280 nm) of each sample.

The amplified nucleic acid contained in the analyte sample and the control sample was subjected to fragmentation and biotin labeling using FL-Ovation (trademark) cDNA Biotin Module V2 (NuGEN).

### Microarray Analysis

### (1) Contact of Sample with Microarray

The analyte sample and the control sample prepared as described above were brought into contact with a microarray, GeneChip (registered trademark) Human Promoter 1.0R Array (Affymetrix, Inc.) and subjected to hybridization to the probes of the microarray using Hybridization Oven 645 (Affymetrix, Inc.). One piece of the same microarray was used for each of the analyte sample and the control sample. After the contact of each sample, the microarray was stained and washed using Fluidics Station 450 (Affymetrix, Inc.). After the washing, the microarray was mounted on GeneChip® AutoLoader (Affymetrix, Inc.) and scanned (for signal measurement) using GeneChip® Scanner 3000 7G (Affymetrix, Inc.). The result of the scan was processed in the computer work station provided by Affymetrix, Inc., and binary files (CEL files) of experimental data were obtained. The procedure from the hybridization to the experimental data acquisition was performed according to the manual provided by Affymetrix, Inc.

### (2) Data Analysis by Conventional Method (TAS)

Based on the CEL files obtained from the analyte sample and the control sample, signal measurement values and a significance probability were obtained using TAS (Affymetrix, Inc.). The resulting significance probability was visualized in the form of a bar graph using a standard browser, Integrated Genome Browser (IGB) (Affymetrix, Inc.). The significance probability displayed in IGB is converted by the formula: (converted value)=-10log₁₀(significance probability), and a converted value of 20 or more suggests that the target nucleic acid should be significantly detected.

In this example, however, the TAS analysis of the genomic DNA in the promoter region of the GSTP1 gene known to be modified by methylation in MCF-7 cells was not able to significantly detect the methylated DNA from the analyte sample derived from MCF-7 cells as well as the samples derived from HMEC and MB-MDA231 cells.

### (3) Data Analysis by the Method of the Invention

The microarray data was analyzed by the method of the invention. The analysis was performed using a general computer.

First, the data shown below was input into the computer.

"CG" as a base sequence to which the nucleic acid-binding protein binds.

The base sequences of the probes mounted on Human Promoter 1.0R Array.

The base sequence of human genomic DNA.

CEL files obtained from the analyte sample and the control sample.

"TACC" which is the recognition sequence of the restriction enzyme Msel.

"300" bases as the length of the Msel restriction enzyme DNA fragments.

The base sequences of the probes mounted on Human Promoter 1.0R Array were obtained from the Affymetrix web page (http://www.affymetrix.com/Auth/analysis/downloads/lf/tiling /Hs-PromPR-v02-3-NCBIv36.bpmap.zip). The base sequence of human genomic sequence was obtained from the NCBI ftp site (ftp://ftp.ncbi.nih.gov/genomes/H_sapiens/April_14_2003).

Data on the base sequences of the DNA fragments obtained by the Msel restriction enzyme treatment of human genomic DNA was obtained from the base sequence of human genomic DNA and the recognition sequence "TACC" of the restriction enzyme Msel. DNA fragments having a base sequence of 300 bases or more were then extracted from "300" bases as the length of the Msel restriction DNA fragments and the base sequences of the resulting DNA fragments. The base sequences of "CG"-free DNA fragments were also extracted from "CG" as the base sequence to which the nucleic acid-binding protein binds and from the extracted DNA fragments having a base sequence of 300 bases or more. Hereinafter, the "base sequences of "CG"-free DNA fragments of 300 bases or more" are also referred to as "fragment sequences."

"CG" sequence-free probes were obtained from "CG" as the base sequence to which the nucleic acid-binding protein binds and from the base sequences of the probes mounted on Human Promoter 1.0R Array. Probes complementary to the fragment sequences were then extracted as probes for correction (correction probes) from the obtained "CG" sequence-free probes and the fragment sequences. The probes other than the correction probes were used as probes for detection (detection probes).

Using Cell File Conversion Tool, signal measurement values were obtained from the CEL files, which were obtained from the analyte sample and the control sample. Cell File Conversion Tool was obtained from the Yvert Lab web page (http://www.ens-lyon.fr/LBMC/gisv/index.php?option=com_content&task=view&id= 37&Itemid=27). First analyte signal measurement values obtained from the detection probes of Human Promoter 1.0R Array in contact with the analyte sample were extracted from the obtained signal measurement values and the information about the detection probes. First control signal measurement values obtained from the detection probes of Human Promoter 1.0R Array in contact with the control sample were also extracted in a similar manner. Second analyte signal measurement values obtained from the correction probes of Human Promoter 1.0R Array in contact with the analyte sample were also extracted from the obtained signal measurement values and the information about the correction probes. Second control signal measurement values obtained from the correction probes of Human Promoter 1.0R Array in contact with the control sample were also extracted in a similar manner.

The mode of the second analyte signal measurement values was calculated as an analyte background value. The mode of the second control signal measurement values was calculated as a control background value.

Analyte correction values were then calculated by subtracting the analyte background value from the first analyte signal measurement values. Control correction values were also calculated by subtracting the control background value from the first control signal measurement values.

When the calculated analyte correction value was negative, the analyte correction value was assumed to be 0. Non-negative analyte correction values were used as they were. In a similar manner, when the calculated control correction value was negative, the control correction value was assumed to be 0. Non-negative control correction values were used as they were.

The S/N ratio after the background correction was calculated by dividing the analyte correction value by the control correction value. However, when the control correction value was 0, the S/N ratio was not calculated for the corresponding probe. The S/N ratio in the case of no background correction was also calculated by dividing the first analyte signal measurement value by the first control signal measurement value.

Significance probabilities were also calculated from the analyte correction values and the control correction values by the Wilcoxon signed-rank test.

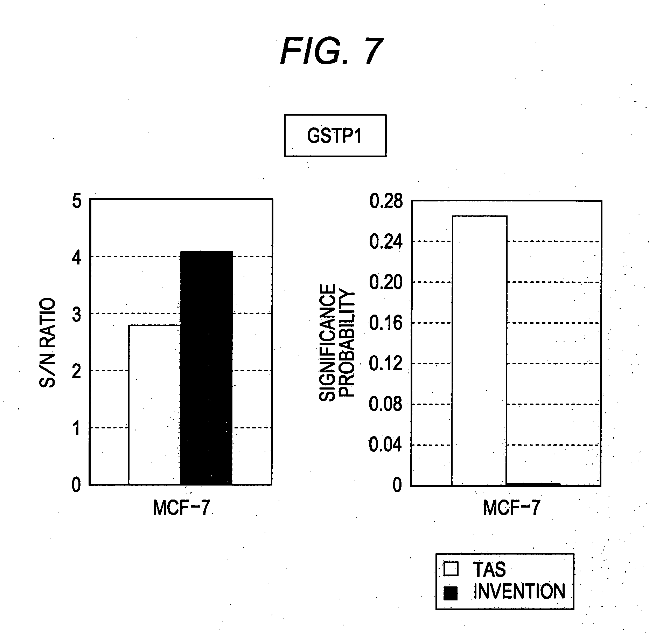

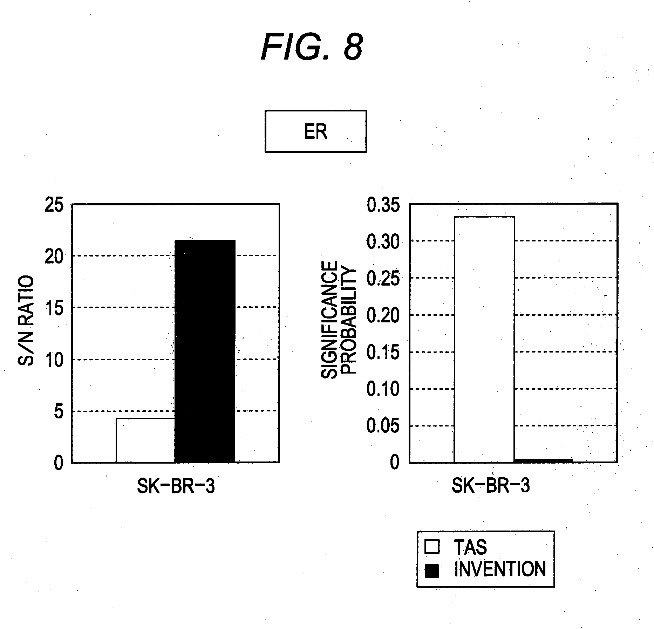

Figs. 7 and 8 are graphs showing the results of the analysis (S/N ratios and significance probabilities) of the analyte samples from MCF-7 and SK-BR-3 cells, respectively. Fig. 7 is a graph showing the average S/N ratio and the average significance probability with respect to 10 consecutive probes for the promoter region of the GSTP1 gene. Fig. 8 is a graph showing the average S/N ratio and the average significance probability with respect to 7 consecutive probes for the promoter region of the ER gene.

The average significance probability calculated according to the method of the invention was 0.01 or less with respect to any of the promoter regions.

From Figs. 7 and 8, it has been found that as compared with conventional TAS analysis, the method of the invention improves the S/N ratio, significantly reduces the significance probability and makes possible high-accuracy detection of methylated genes.

### Example 2

A plurality of quality check control probes for Poly-A control and hybridization control are placed on GeneChip (registered trademark) Human Promoter 1.0R Array (Affymetrix, Inc.) These are external control probes, which are placed on the fact that they do not hybridize to animal-derived nucleic acids.

Thus, the method of the invention was applied to the Poly-A control probes and the hybridization control probes. Specifically, probes not containing any CpG sequence to which a nucleic acid-binding protein (anti-methylated cytosine antibody) binds were extracted from these control probes, and it was examined whether or not the use of them as correction probes made possible higher-accuracy detection of a CpG sequence-containing target nucleic acid.

Analyte and control samples were prepared from MCF-7 cells as in Example 1. The analyte and control samples were then brought into contact with GeneChip (registered trademark) Human Promoter 1.0R Array (Affymetrix, Inc.) as in Example 1, so that each signal measurement value was obtained.

Poly-A control probes and hybridization control probes were extracted from the probes of each microarray, and CpG sequence-free probes (hereinafter also referred to as "correction probes C") were further extracted from these probes. For comparison, control probes consisting of Poly-A control probes and hybridization control probes were named correction probes D.

Signal measurement values were obtained from correction probes C on the microarray in contact with the analyte sample, and the mode of the signal measurement values were obtained and named background value C. Signal measurement values were also obtained from correction probes D, and the mode of them was also obtained and named background value D.

Analyte correction values C were calculated by subtracting background value C from first analyte signal measurement values. Analyte correction values D were also calculated by subtracting background value D from first analyte signal measurement values.

Control correction values C and D were also calculated in the same manner as described above with respect to the microarray in contact with the control sample.

Significance probabilities were calculated by the Wilcoxon signed-rank test using the calculated analyte correction value A and control correction value A as a pair of samples and using the calculated analyte correction value B and control correction value B as a pair of samples, respectively.

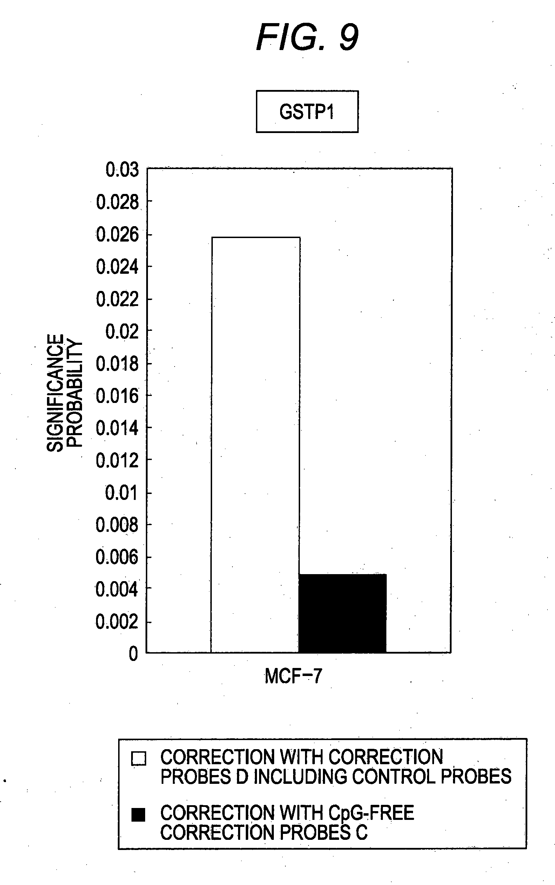

Fig. 9 shows the significance probabilities obtained in cases where background correction was performed using the correction probes C and D, respectively. The graph shows the average of significance probabilities obtained using 10 consecutive probes for GSTP1.

Fig. 9 shows that the significance probability obtained by performing background correction with CpG sequence-containing correction probes D is higher than 0.008, while the significance probability obtained by performing background correction with CpG sequence-free correction probes C is lower than 0.008, which means that higher-accuracy detection is possible by the background correction with correction probes C.

If microarray-based target nucleic acid detection is used to find drug targets or to diagnose diseases, the results of the detection should have high reliability. Therefore, the significance probability obtained from data should preferably be as low as possible.

Thus, it has been demonstrated that background correction performed using CpG sequence-free correction probes extracted from probes not complementary to the nucleic acid in the analyte sample is more useful than background correction performed using the probes not complementary to the nucleic acid in the analyte sample.

## Claims

1. A method of detecting, with a microarray, a target nucleic acid from an organism containing a recognition sequence which is a base sequence to which a nucleic acid-binding protein is known to specifically bind, comprising steps of:
(1) contacting an analyte sample potentially containing the target nucleic acid with a microarray having a plurality of probes placed on a substrate of the microarray;
(2) selecting a detection probe from the plurality of probes, wherein said detection probe has a sequence complementary to said recognition sequence or having a sequence complementary to a region within 300 bases from the recognition sequence in the target nucleic acid sequence, and is capable of hybridizing to the target nucleic acid;
(3) selecting aprobe from the plurality of probes, which does not contain any sequence complementary to the recognition sequence and hybridizes to a genomic DNA of said organism that does not contain the recognition sequence in the region within 300 bases from the probe hybridizing region, as a correction probe;
(4) obtaining a first signal measurement value and a second signal measurement value respectively by measuring a first signal obtained from the detection probe, selected in step (2), and a second signal obtained from the correction probe, selected in step (3), after the contacting step;
(5) obtaining a background value based on the second signal measurement value;
(6) obtaining a first signal correction value by correcting the first signal measurement value with the background value; and
(7) detecting the target nucleic acid contained in the analyte sample based on the correction value.

2. The method of claim 1, wherein the microarray is a commercially available microarray.

3. The method according to claims 1 or 2, wherein the microarray has a plurality of correction probes, and the background value is the mode of second signal measurement values obtained from the plurality of correction probes.

4. The method according to any one of claims 1 to 3, wherein the step (7) is a step of detecting the target nucleic acid from the analyte sample based on the result of a comparison between the correction value and a predetermined threshold value.

5. A method of detecting, with a microarray, a target nucleic acid from an organism containing a recognition sequence which is a base sequence to which a nucleic acid-binding protein is known to specifically bind, comprising steps of:
(1) contacting an analyte sample potentially containing the target nucleic acid with a microarray A having a plurality of probes placed on a substrate of the microarray; and
contacting a control sample with no possibility of containing the target nucleic acid with a microarray B having the same plurality of probes placed on a substrate of the microarray as the microarray A;
(2) selecting a detection probe from the plurality of probes present in the microarrays A and B, wherein said detection probe has a sequence complementary to said recognition sequence or having a sequence complementary to a region within 300 bases from the recognition sequence in the target nucleic acid sequence, and is capable of hybridizing to the target nucleic acid;
(3) selecting a probe from the plurality of probes present in the microarrays A and B, which does not contain any sequence complementary to the recognition sequence and hybridizes to a genomic DNA of said organism that does not contain the recognition sequence in the region within 300 bases from the probe hybridizing region, as a correction probe;
(4) obtaining a first analyte signal measurement value and a second analyte signal measurement value respectively by measuring a first analyte signal obtained from the detection probe of the microarray A, selected in step (2), and a second analyte signal obtained from the correction probe of the microarray A, selected in step (3), and
obtaining a first control signal measurement value and a second control signal measurement value respectively by measuring a first control signal obtained from the detection probe of the microarray B, selected in step (2), and a second control signal obtained from the correction probe of the microarray B, selected in step (3),
after the contacting step;
(5) obtaining an analyte background value and a control background value respectively, based on the second analyte signal measurement value and the second control signal measurement value;
(6) obtaining a analyte correction value for the first analyte signal by correcting the first analyte signal measurement value with the analyte background value, and obtaining a control correction value for the first control signal by correcting the first control signal measurement value with the control background value;
(7) obtaining an analysis value based on the analyte correction value and the control correction value; and
(8) detecting the target nucleic acid contained in the analyte sample, based on the analysis value.

6. The method according to claim 5, wherein the microarrays A and B each have a plurality of correction probes,
the analyte background value is the mode of second analyte signal measurement values obtained from the plurality of correction probes of the microarray A, and
the control background value is the mode of second control signal measurement values obtained from the plurality of correction probes of the microarray B.

7. The method according to any one of claims 5 or 6, wherein the microarrays A and B each have a plurality of detection probes,
analyte correction values are obtained by subtracting the analyte background value from first analyte signal measurement values obtained from the plurality of detection probes of the microarray A,
control correction values are obtained by subtracting the control background value from first control signal measurement values obtained from the plurality of detection probes of the microarray B, and
the analysis value is a significance probability.

8. The method according to any one of claims 5 to 7, wherein the step (8) is a step of detecting the target nucleic acid from the analyte sample based on the result of a comparison between the analysis value and a predetermined threshold value.

9. The method according to any one of claims 1 to 8, further comprising steps of:
(i) obtaining the target nucleic acid from a biological sample by an immunoprecipitation method; and
(ii) amplifying the target nucleic acid by a nucleic acid amplification method to prepare an analyte sample.

10. The method according to claim 9, wherein the step (ii) further comprises a step of subjecting the nucleic acid amplified by the nucleic acid amplification method to fluorescent labeling, and the signal obtained from the probe placed on the microarray is fluorescence intensity.

11. The method according to any one of claims 1 to 10, wherein the base sequence is a CpG sequence.

12. A computer program product for enabling a computer to detect, with a microarray having a plurality of probes placed on a substrate of the microarray, a target nucleic acid from an organism, containinga recognition sequence, which is a base sequence to which a nucleic acid-binding protein is known to specifically bind, comprising:
a computer readable medium; and
software instructions, on the computer readable medium, for enabling the computer to perform predetermined operations comprising:
(1) receiving the recognition sequence and the base sequences of the plurality of probes placed on the microarray in contact with the analyte sample potentially containing the target nucleic acid;
(2) selecting a detection probe from the plurality of probes, wherein said detection probe has a sequence complementary to said recognition sequence or a sequence complementary to a region within 300 bases from the recognition sequence in the target nucleic acid sequence, and is capable of hybridizing to the target nucleic acid;
(3) selecting a probe from the plurality of probes, which does not contain any sequence complementary to the recognition sequence and hybridizes to a genomic DNA of said organism that does not contain the recognition sequence in the region within 300 bases from the probe hybridizing region, as a correction probe;
(4) obtaining a first signal measurement value derived from a detection probe, selected in step (2)and placed on a microarray in contact with an analyte sample potentially containing the target nucleic acid, and
a second signal measurement value derived from a correction probe, selected in step (3), placed on the microarray;
(5) obtaining a background value based on the second signal measurement value;
(6) obtaining a first signal correction value by correcting the first signal measurement value with the background value; and
(7) outputting the first signal correction value.

13. The computer program product according to claim 12, wherein
the microarray has a plurality of correction probes, and
the background value is the mode of second signal measurement values obtained from the plurality of correction probes.

14. The computer program product according to any one of claims 12 or 13, wherein
the microarray has a plurality of detection probes, and
correction values are obtained by correcting, with the background value, first signal measurement values obtained from the plurality of detection probes of the microarray.

15. A computer program product for enabling a computer to detect, with a microarray having a plurality of probes placed on a substrate of the microarray, a target nucleic acid from an organism containing a recognition sequence which is a base sequence to which a nucleic acid-binding protein is known to specifically bind, comprising:
a computer readable medium; and
software instructions, on the computer readable medium, for enabling the computer to perform predetermined operations comprising:
(1) receiving the recognition sequence and the base sequences of the plurality of probes which are present in a microarray A placed in contact with an analyte sample potentially containing the target nucleic acid, and which are present in a microarray B placed in contact with a control sample having no possibility of containing the target nucleic acid
(2) selecting a detection probe from the plurality of probes present in the microarrays A and B, wherein said detection probe has asequence complementary to said recognition sequence or a sequence complementary to a region within 300 bases from the recognition sequence in the target nucleic acid sequence, and is capable of hybridizing to the target nucleic acid;
(3) selecting aprobe from the plurality of probes present in the microarrays A and B, which does not contain any sequence complementary to the recognition sequence and hybridizes to a genomic DNA of said organism that does not contain the recognition sequence in the region within 300 bases from the probe hybridizing region, as a correction probe;
(4) obtaining a first analyte signal measurement value derived from a detection probe that is present in a microarray A, selected in step (2) in contact with an analyte sample potentially containing the target nucleic acid, and
a second analyte signal measurement value derived from a correction probe that is present in the microarray A, selected in step (3),
obtaining a first control signal measurement value derived from a detection probe present in a microarray B, selected in step (2) in contact with a control sample, and
obtaining a second control signal measurement value derived from a correction probe that is present in the microarray B, selected in step (3) ;
(5) obtaining an analyte background value and a control background value, respectively, based on the second analyte signal measurement value and the second control signal measurement value;
(6) obtaining a analyte correction value for the first analyte signal by correcting the first analyte signal measurement value with the analyte background value, and
a control correction value for the first control signal by correcting the first control signal measurement value with the control background value;
(7) obtaining an analysis value based on the analyte correction value and the control correction value; and
(8) outputting the analysis value.

16. The computer program product according to claim 15, wherein
the microarrays A and B each have a plurality of correction probes,
the analyte background value is the mode of second analyte signal measurement values obtained from the plurality of correction probes of the microarray A, and
the control background value is the mode of second control signal measurement values obtained from the plurality of correction probes of the microarray B.

17. The computer program product according to any one of claims 15 or 16, wherein
the microarrays A and B each have a plurality of detection probes,
analyte correction values are obtained by subtracting the analyte background value from first analyte signal measurement values obtained from the plurality of detection probes of the microarray A,
control correction values are obtained by subtracting the control background value from first control signal measurement values obtained from the plurality of detection probes of the microarray B, and
the analysis value is a significance probability.

## Patentansprüche

1. Verfahren zum Nachweis, mit einem Mikroarray, einer Zielnukleinsäure aus einem Organismus, die eine Erkennungssequenz enthält, bei der es sich um eine Basensequenz handelt, von der bekannt ist, dass ein nukleinsäurebindendes Protein spezifisch daran bindet, umfassend die Schritte:
(1) Inkontaktbringen einer möglicherweise die Zielnukleinsäure enthaltenden Analytprobe mit einem Mikroarray mit mehreren auf einem Substrat des Mikroarrays platzierten Sonden;
(2) Auswählen einer Nachweissonde aus den mehreren Sonden, wobei die Nachweissonde eine zur Erkennungssequenz komplementäre Sequenz aufweist oder mit einer zu einem Bereich innerhalb von 300 Basen von der Erkennungssequenz in der Zielnukleinsäuresequenz komplementären Sequenz und in der Lage ist, an die Zielnukleinsäure zu hybridisieren;
(3) Auswählen einer Sonde aus den mehreren Sonden, die keine zur Erkennungssequenz komplementäre Sequenz enthält und an eine genomische DNA des Organismus hybridisiert, die nicht die Erkennungssequenz in dem Bereich innerhalb von 300 Basen vom Sondenhybridisierungsbereich enthält, als Korrektursonde;
(4) Erhalten eines ersten Signalmesswerts bzw. eines zweiten Signalmesswerts durch Messen eines von der Nachweissonde, ausgewählt in Schritt (2), erhaltenen ersten Signals bzw. eines von der Korrektursonde, ausgewählt in Schritt (3), erhaltenen zweiten Signals, nach dem Kontaktierungsschritt;
(5) Erhalten eines Hintergrundwerts auf der Grundlage des zweiten Signalmesswerts;
(6) Erhalten eines ersten Signalkorrekturwerts durch Korrigieren des ersten Signalmesswerts mit dem Hintergrundwert; und
(7) Nachweisen der in der Analytprobe enthaltenen Zielnukleinsäure auf der Grundlage des Korrekturwerts.

2. Verfahren gemäß Anspruch 1, wobei es sich bei dem Mikroarray um ein im Handel erhältliches Mikroarray handelt.

3. Verfahren nach Anspruch 1 oder 2, wobei das Mikroarray mehrere Korrektursonden aufweist und es sich bei dem Hintergrundwert um den Modus von von den mehreren Korrektursonden erhaltenen zweiten Signalmesswerten handelt.

4. Verfahren nach einem der Ansprüche 1 bis 3, wobei es sich bei Schritt (7) um einen Schritt des Nachweisens der Zielnukleinsäure aus der Analytprobe auf der Grundlage des Ergebnisses eines Vergleichs zwischen dem Korrekturwert und einem vorbestimmten Schwellenwert handelt.

5. Verfahren zum Nachweis, mit einem Mikroarray, einer Zielnukleinsäure aus einem Organismus, die eine Erkennungssequenz enthält, bei der es sich um eine Basensequenz handelt, von der bekannt ist, dass ein nukleinsäurebindendes Protein spezifisch daran bindet, umfassend die Schritte:
(1) Inkontaktbringen einer möglicherweise die Zielnukleinsäure enthaltenden Analytprobe mit einem Mikroarray A mit mehreren auf einem Substrat des Mikroarrays platzierten Sonden; und
Inkontaktbringen einer Kontrollprobe, bei der keine Möglichkeit besteht, dass sie die Zielnukleinsäure enthält, mit einem Mikroarray B mit den gleichen mehreren auf einem Substrat des Mikroarrays platzierten Sonden wie Mikroarray A;
(2) Auswählen einer Nachweissonde aus den in den Mikroarrays A und B vorliegenden mehreren Sonden, wobei die Nachweissonde eine zur Erkennungssequenz komplementäre Sequenz aufweist oder mit einer zu einem Bereich innerhalb von 300 Basen von der Erkennungssequenz in der Zielnukleinsäuresequenz komplementären Sequenz und in der Lage ist, an die Zielnukleinsäure zu hybridisieren;
(3) Auswählen einer Sonde aus den in den Mikroarrays A und B vorliegenden mehreren Sonden, die keine zur Erkennungssequenz komplementäre Sequenz enthält und an eine genomische DNA des Organismus hybridisiert, die nicht die Erkennungssequenz in dem Bereich innerhalb von 300 Basen vom Sondenhybridisierungsbereich enthält, als Korrektursonde;
(4) Erhalten eines ersten Analytsignalmesswerts bzw. eines zweiten Analytsignalmesswerts durch Messen eines von der Nachweissonde des Mikroarrays A, ausgewählt in Schritt (2), erhaltenen ersten Analytsignals bzw. eines von der Korrektursonde des Mikroarrays A, ausgewählt in Schritt (3), erhaltenen zweiten Analytsignals; und
Erhalten eines ersten Kontrollsignalmesswerts bzw. eines zweiten Kontrollsignalmesswerts durch Messen eines von der Nachweissonde des Mikroarrays B, ausgewählt in Schritt (2), erhaltenen ersten Kontrollsignals bzw. eines von der Korrektursonde des Mikroarrays B, ausgewählt in Schritt (3), erhaltenen zweiten Kontrollsignals,
nach dem Kontaktierungsschritt;
(5) Erhalten eines Analythintergrundwerts bzw. eines Kontrollhintergrundwerts auf der Grundlage des zweiten Analytsignalmesswerts bzw. des zweiten Kontrollsignalmesswerts;
(6) Erhalten eines Analytkorrekturwerts für das erste Analytsignal durch Korrigieren des ersten Analytsignalmesswerts mit dem Analythintergrundwert und
Erhalten eines Kontrollkorrekturwerts für das erste Kontrollsignal durch Korrigieren des ersten Kontrollsignalmesswerts mit dem Kontrollhintergrundwert;
(7) Erhalten eines Analysewerts auf der Grundlage des Analytkorrekturwerts und des Kontrollkorrekturwerts; und
(8) Nachweisen der in der Analytprobe enthaltenen Zielnukleinsäure auf der Grundlage des Analysewerts.

6. Verfahren nach Anspruch 5, wobei die Mikroarrays A und B jeweils mehrere Korrektursonden aufweisen,
es sich bei dem Analythintergrundwert um den Modus von von den mehreren Korrektursonden des Mikroarrays A erhaltenen zweiten Analytsignalmesswerten handelt und
es sich bei dem Kontrollhintergrundwert um den Modus von von den mehreren Korrektursonden des Mikroarrays B erhaltenen zweiten Kontrollsignalmesswerten handelt.

7. Verfahren nach einem der Ansprüche 5 oder 6, wobei die Mikroarrays A und B jeweils mehrere Nachweissonden aufweisen,
Analytkorrekturwerte durch Subtrahieren des Analythintergrundwerts von von den mehreren Nachweissonden des Mikroarrays A erhaltenen ersten Analytsignalmesswerten erhalten werden,
Kontrollkorrekturwerte durch Subtrahieren des Kontrollhintergrundwerts von von den mehreren Nachweissonden des Mikroarrays B erhaltenen ersten Kontrollsignalmesswerten erhalten werden und
es sich bei dem Analysewert um eine Signifikanzwahrscheinlichkeit handelt.

8. Verfahren nach einem der Ansprüche 5 bis 7, wobei es sich bei Schritt (8) um einen Schritt des Nachweisens der Zielnukleinsäure aus der Analytprobe auf der Grundlage des Ergebnisses eines Vergleichs zwischen dem Analysewert und einem vorbestimmten Schwellenwert handelt.

9. Verfahren nach einem der Ansprüche 1 bis 8, ferner umfassend die Schritte:
(i) Erhalten der Zielnukleinsäure aus einer biologischen Probe mit einem Immunpräzipitationsverfahren; und
(ii) Amplifizieren der Zielnukleinsäure mit einem Nukleinsäureamplifikationsverfahren zur Herstellung einer Analytprobe.

10. Verfahren nach Anspruch 9, wobei der Schritt (ii) ferner einen Schritt umfasst, bei dem die mit dem Nukleinsäureamplifikationsverfahren amplifizierte Nukleinsäure einer Fluoreszenzmarkierung unterzogen wird, und es sich bei dem von der auf dem Mikroarray platzierten Sonde erhaltenen Signal um Fluoreszenzintensität handelt.

11. Verfahren nach einem der Ansprüche 1 bis 10, wobei es sich bei der Basensequenz um eine CpG-Sequenz handelt.

12. Computerprogrammprodukt, um einem Computer das Nachweisen, mit einem Mikroarray mit mehreren auf einem Substrat des Mikroarrays platzierten Sonden, einer Zielnukleinsäure aus einem Organismus, die eine Erkennungssequenz enthält, bei der es sich um eine Basensequenz handelt, von der bekannt ist, dass ein nukleinsäurebindendes Protein spezifisch daran bindet, zu ermöglichen, umfassend:
einen computerlesbaren Datenträger; und
Software-Anweisungen, auf dem computerlesbaren Datenträger, um dem Computer das Durchführen vorbestimmter Arbeiten zu ermöglichen, umfassend:
(1) Empfangen der Erkennungssequenz und der Basensequenzen der mehreren auf dem Mikroarray in Kontakt mit der möglicherweise die Zielnukleinsäure enthaltenden Analytprobe platzierten Sonden;
(2) Auswählen einer Nachweissonde aus den mehreren Sonden, wobei die Nachweissonde eine zur Erkennungssequenz komplementäre Sequenz oder eine zu einem Bereich innerhalb von 300 Basen von der Erkennungssequenz in der Zielnukleinsäuresequenz komplementäre Sequenz aufweist und in der Lage ist, an die Zielnukleinsäure zu hybridisieren;
(3) Auswählen einer Sonde aus den mehreren Sonden, die keine zur Erkennungssequenz komplementäre Sequenz enthält und an eine genomische DNA des Organismus hybridisiert, die nicht die Erkennungssequenz in dem Bereich innerhalb von 300 Basen vom Sondenhybridisierungsbereich enthält, als Korrektursonde;
(4) Erhalten eines von einer Nachweissonde, ausgewählt in Schritt (2) und auf einem Mikroarray in Kontakt mit einer möglicherweise die Zielnukleinsäure enthaltenden Analytprobe platziert, stammenden ersten Signalmesswerts und
eines von einer Korrektursonde, ausgewählt in Schritt (3), platziert auf dem Mikroarray, stammenden zweiten Signalmesswerts;
(5) Erhalten eines Hintergrundwerts auf der Grundlage des zweiten Signalmesswerts;
(6) Erhalten eines ersten Signalkorrekturwerts durch Korrigieren des ersten Signalmesswerts mit dem Hintergrundwert; und
(7) Ausgeben des ersten Signalkorrekturwerts.

13. Computerprogrammprodukt nach Anspruch 12, wobei
das Mikroarray mehrere Korrektursonden aufweist und
es sich bei dem Hintergrundwert um den Modus von von den mehreren Korrektursonden erhaltenen zweiten Signalmesswerten handelt.

14. Computerprogrammprodukt nach einem der Ansprüche 12 oder 13, wobei
das Mikroarray mehrere Nachweissonden aufweist und
Korrekturwerte durch Korrigieren, mit dem Hintergrundwert, von den mehreren Nachweissonden des Mikroarrays erhaltener erster Signalmesswerte erhalten werden.

15. Computerprogrammprodukt, um einem Computer das Nachweisen, mit einem Mikroarray mit mehreren auf einem Substrat des Mikroarrays platzierten Sonden, einer Zielnukleinsäure aus einem Organismus, die eine Erkennungssequenz enthält, bei der es sich um eine Basensequenz handelt, von der bekannt ist, dass ein nukleinsäurebindendes Protein spezifisch daran bindet, zu ermöglichen, umfassend:
einen computerlesbaren Datenträger; und
Software-Anweisungen, auf dem computerlesbaren Datenträger, um dem Computer das Durchführen vorbestimmter Arbeiten zu ermöglichen, umfassend:
(1) Empfangen der Erkennungssequenz und der Basensequenzen der mehreren Sonden, die in einem in Kontakt mit einer möglicherweise die Zielnukleinsäure enthaltenden Analytprobe platzierten Mikroarray A vorliegen und die in einem in Kontakt mit einer Kontrollprobe, bei der keine Möglichkeit besteht, dass sie die Zielnukleinsäure enthält, platzierten Mikroarray B vorliegen;
(2) Auswählen einer Nachweissonde aus den in den Mikroarrays A und B vorliegenden mehreren Sonden, wobei die Nachweissonde eine zur Erkennungssequenz komplementäre Sequenz oder eine zu einem Bereich innerhalb von 300 Basen von der Erkennungssequenz in der Zielnukleinsäuresequenz komplementäre Sequenz aufweist und in der Lage ist, an die Zielnukleinsäure zu hybridisieren;
(3) Auswählen einer Sonde aus den in den Mikroarrays A und B vorliegenden mehreren Sonden, die keine zur Erkennungssequenz komplementäre Sequenz enthält und an eine genomische DNA des Organismus hybridisiert, die nicht die Erkennungssequenz in dem Bereich innerhalb von 300 Basen vom Sondenhybridisierungsbereich enthält, als Korrektursonde;
(4) Erhalten eines von einer Nachweissonde, die in einem Mikroarray A vorliegt, ausgewählt in Schritt (2) in Kontakt mit einer möglicherweise die Zielnukleinsäure enthaltenden Analytprobe, stammenden ersten Analytsignalmesswerts und eines von einer Korrektursonde, die im Mikroarray A vorliegt, ausgewählt in Schritt (3), stammenden zweiten Analytsignalmesswerts;
Erhalten eines von einer in einem Mikroarray B vorliegenden Nachweissonde, ausgewählt in Schritt (2) in Kontakt mit einer Kontrollprobe, stammenden ersten Kontrollsignalmesswerts und
Erhalten eines von einer Korrektursonde, die im Mikroarray B vorliegt, ausgewählt in Schritt (3), stammenden zweiten Kontrollsignalmesswerts;
(5) Erhalten eines Analythintergrundwerts bzw. eines Kontrollhintergrundwerts auf der Grundlage des zweiten Analytsignalmesswerts bzw. des zweiten Kontrollsignalmesswerts;
(6) Erhalten eines Analytkorrekturwerts für das erste Analytsignal durch Korrigieren des ersten Analytsignalmesswerts mit dem Analythintergrundwert und
eines Kontrollkorrekturwerts für das erste Kontrollsignal durch Korrigieren des ersten Kontrollsignalmesswerts mit dem Kontrollhintergrundwert;
(7) Erhalten eines Analysewerts auf der Grundlage des Analytkorrekturwerts und des Kontrollkorrekturwerts; und
(8) Ausgeben des Analysewerts.

16. Computerprogrammprodukt nach Anspruch 15, wobei
die Mikroarrays A und B jeweils mehrere Korrektursonden aufweisen,
es sich bei dem Analythintergrundwert um den Modus von von den mehreren Korrektursonden des Mikroarrays A erhaltenen zweiten Analytsignalmesswerten handelt und
es sich bei dem Kontrollhintergrundwert um den Modus von von den mehreren Korrektursonden des Mikroarrays B erhaltenen zweiten Kontrollsignalmesswerten handelt.

17. Computerprogrammprodukt nach einem der Ansprüche 15 oder 16, wobei
die Mikroarrays A und B jeweils mehrere Nachweissonden aufweisen,
Analytkorrekturwerte durch Subtrahieren des Analythintergrundwerts von von den mehreren Nachweissonden des Mikroarrays A erhaltenen ersten Analytsignalmesswerten erhalten werden,
Kontrollkorrekturwerte durch Subtrahieren des Kontrollhintergrundwerts von von den mehreren Nachweissonden des Mikroarrays B erhaltenen ersten Kontrollsignalmesswerten erhalten werden und
es sich bei dem Analysewert um eine Signifikanzwahrscheinlichkeit handelt.

## Revendications

1. Procédé de détection, avec un microréseau, d'un acide nucléique cible issu d'un organisme contenant une séquence de reconnaissance qui est une séquence de bases à laquelle une protéine de liaison aux acides nucléiques est connue pour se lier spécifiquement, comprenant les étapes consistant à :
(1) mettre un échantillon d'analyte contenant potentiellement l'acide nucléique cible en contact avec un microréseau ayant une pluralité de sondes placées sur un substrat du microréseau ;
(2) sélectionner une sonde de détection parmi la pluralité de sondes, ladite sonde de détection ayant une séquence complémentaire de ladite séquence de reconnaissance ou ayant une séquence complémentaire d'une région à moins de 300 bases de la séquence de reconnaissance dans la séquence d'acide nucléique cible, et étant susceptible de s'hybrider avec l'acide nucléique cible ;
(3) sélectionner parmi la pluralité de sondes une sonde qui ne contient aucune séquence complémentaire de la séquence de reconnaissance et s'hybride avec un ADN génomique dudit organisme qui ne contient pas la séquence de reconnaissance dans la région à moins de 300 bases de la région d'hybridation à la sonde, en tant que sonde de correction ;
(4) obtenir respectivement une première valeur de mesure de signal et une deuxième valeur de mesure de signal en mesurant un premier signal obtenu à partir de la sonde de détection, sélectionnée à l'étape (2), et un deuxième signal obtenu à partir de la sonde de correction, sélectionnée à l'étape (3), après l'étape de mise en contact ;
(5) obtenir une valeur de bruit de fond basée sur la deuxième valeur de mesure de signal ;
(6) obtenir une première valeur de correction de signal en corrigeant la première valeur de mesure de signal par la valeur de bruit de fond ; et
(7) détecter l'acide nucléique cible contenu dans l'échantillon d'analyte sur la base de la valeur de correction.

2. Procédé selon la revendication 1, dans lequel le microréseau est un microréseau disponible dans le commerce.

3. Procédé selon la revendication 1 ou 2, dans lequel le microréseau a une pluralité de sondes de correction, et la valeur de bruit de fond est le mode des deuxièmes valeurs de mesure de signal obtenues à partir de la pluralité de sondes de correction.

4. Procédé selon l'une quelconque des revendications 1 à 3, dans lequel l'étape (7) est une étape de détection de l'acide nucléique cible issu de l'échantillon d'analyte sur la base du résultat d'une comparaison entre la valeur de correction et une valeur seuil prédéterminée.

5. Procédé de détection, avec un microréseau, d'un acide nucléique cible issu d'un organisme contenant une séquence de reconnaissance qui est une séquence de bases à laquelle une protéine de liaison aux acides nucléiques est connue pour se lier spécifiquement, comprenant les étapes consistant à :
(1) mettre un échantillon d'analyte contenant potentiellement l'acide nucléique cible en contact avec un microréseau A ayant une pluralité de sondes placées sur un substrat du microréseau ; et
mettre un échantillon témoin sans aucune possibilité de contenir l'acide nucléique cible en contact avec un microréseau B ayant la même pluralité de sondes placées sur un substrat du microréseau que le microréseau A ;
(2) sélectionner une sonde de détection parmi la pluralité de sondes présentes dans les microréseaux A et B, ladite sonde de détection ayant une séquence complémentaire de ladite séquence de reconnaissance ou ayant une séquence complémentaire d'une région à moins de 300 bases de la séquence de reconnaissance dans la séquence d'acide nucléique cible, et étant susceptible de s'hybrider avec l'acide nucléique cible ;
(3) sélectionner parmi la pluralité de sondes présentes dans les microréseaux A et B une sonde qui ne contient aucune séquence complémentaire de la séquence de reconnaissance et s'hybride avec un ADN génomique dudit organisme qui ne contient pas la séquence de reconnaissance dans la région à moins de 300 bases de la région d'hybridation à la sonde, en tant que sonde de correction ;
(4) obtenir respectivement une première valeur de mesure de signal d'analyte et une deuxième valeur de mesure de signal d'analyte en mesurant un premier signal d'analyte obtenu à partir de la sonde de détection du microréseau A, sélectionnée à l'étape (2), et un deuxième signal d'analyte obtenu à partir de la sonde de correction du microréseau A, sélectionnée à l'étape (3), et
obtenir respectivement une première valeur de mesure de signal de contrôle et une deuxième valeur de mesure de signal de contrôle en mesurant un premier signal de contrôle obtenu à partir de la sonde de détection du microréseau B, sélectionnée à l'étape (2), et un deuxième signal de contrôle obtenu à partir de la sonde de correction du microréseau B, sélectionnée à l'étape (3),
après l'étape de mise en contact ;
(5) obtenir respectivement une valeur de bruit de fond d'analyte et une valeur de bruit de fond de contrôle, basées sur la deuxième valeur de mesure de signal d'analyte et la deuxième valeur de mesure de signal de contrôle ;
(6) obtenir une valeur de correction d'analyte pour le premier signal d'analyte en corrigeant la première valeur de mesure de signal d'analyte par la valeur de bruit de fond d'analyte, et
obtenir une valeur de correction de contrôle pour le premier signal de contrôle en corrigeant la première valeur de mesure de signal de contrôle par la valeur de bruit de fond de contrôle ;
(7) obtenir une valeur d'analyse basée sur la valeur de correction d'analyte et la valeur de correction de contrôle ; et
(8) détecter l'acide nucléique cible contenu dans l'échantillon d'analyte sur la base de la valeur d'analyse.

6. Procédé selon la revendication 5, dans lequel les microréseaux A et B ont chacun une pluralité de sondes de correction,
la valeur de bruit de fond d'analyte est le mode des deuxièmes valeurs de mesure de signal d'analyte obtenues à partir de la pluralité de sondes de correction du microréseau A, et
la valeur de bruit de fond de contrôle est le mode des deuxièmes valeurs de mesure de signal de contrôle obtenues à partir de la pluralité de sondes de correction du microréseau B.

7. Procédé selon l'une quelconque des revendications 5 et 6, dans lequel les microréseaux A et B ont chacun une pluralité de sondes de détection,
les valeurs de correction d'analyte sont obtenues en soustrayant la valeur de bruit de fond d'analyte des premières valeurs de mesure de signal d'analyte obtenues à partir de la pluralité de sondes de détection du microréseau A,
les valeurs de correction de contrôle sont obtenues en soustrayant la valeur de bruit de fond de contrôle des premières valeurs de mesure de signal de contrôle obtenues à partir de la pluralité de sondes de détection du microréseau B, et
la valeur d'analyse est une probabilité de signification.

8. Procédé selon l'une quelconque des revendications 5 à 7, dans lequel l'étape (8) est une étape de détection de l'acide nucléique cible issu de l'échantillon d'analyte sur la base du résultat d'une comparaison entre la valeur d'analyse et une valeur seuil prédéterminée.

9. Procédé selon l'une quelconque des revendications 1 à 8, comprenant en outre les étapes consistant à :
(i) obtenir l'acide nucléique cible à partir d'un échantillon biologique par un procédé d'immunoprécipitation ; et
(ii) amplifier l'acide nucléique cible par un procédé d'amplification d'acide nucléique pour préparer un échantillon d'analyte.

10. Procédé selon la revendication 9, dans lequel l'étape (ii) comprend en outre une étape consistant à soumettre l'acide nucléique amplifié par le procédé d'amplification d'acide nucléique à un marquage fluorescent, et le signal obtenu à partir de la sonde placée sur le microréseau est une intensité de fluorescence.

11. Procédé selon l'une quelconque des revendications 1 à 10, dans lequel la séquence de base est une séquence CpG.

12. Produit-programme informatique destiné à permettre à un ordinateur de détecter, avec un microréseau ayant une pluralité de sondes placées sur un substrat du microréseau, un acide nucléique cible issu d'un organisme contenant une séquence de reconnaissance, qui est une séquence de bases à laquelle une protéine de liaison aux acides nucléiques est connue pour se lier spécifiquement, comprenant :
un support lisible par ordinateur ; et
des instructions logicielles, sur le support lisible par ordinateur, pour permettre à l'ordinateur d'effectuer des opérations prédéterminées comprenant :
(1) la réception de la séquence de reconnaissance et des séquences de base de la pluralité de sondes placées sur le microréseau en contact avec l'échantillon d'analyte contenant potentiellement l'acide nucléique cible ;
(2) la sélection d'une sonde de détection parmi la pluralité de sondes, ladite sonde de détection ayant une séquence complémentaire de ladite séquence de reconnaissance ou une séquence complémentaire d'une région à moins de 300 bases de la séquence de reconnaissance dans la séquence d'acide nucléique cible, et étant susceptible de s'hybrider avec l'acide nucléique cible ;
(3) la sélection parmi la pluralité de sondes d'une sonde qui ne contient aucune séquence complémentaire de la séquence de reconnaissance et s'hybride avec un ADN génomique dudit organisme qui ne contient pas la séquence de reconnaissance dans la région à moins de 300 bases de la région d'hybridation à la sonde, en tant que sonde de correction ;
(4) l'obtention d'une première valeur de mesure de signal à partir d'une sonde de détection, sélectionnée à l'étape (2) et placée sur le microréseau en contact avec un échantillon d'analyte contenant potentiellement l'acide nucléique cible, et
d'une deuxième valeur de mesure de signal dérivée d'une sonde de correction, sélectionnée à l'étape (3), placée sur le microréseau ;
(5) l'obtention d'une valeur de bruit de fond basée sur la deuxième valeur de mesure de signal ;
(6) l'obtention d'une première valeur de correction de signal par correction de la première valeur de mesure de signal avec la valeur de bruit de fond ; et
(7) la sortie de la première valeur de correction de signal.

13. Produit-programme informatique selon la revendication 12, dans lequel
le microréseau a une pluralité de sondes de correction, et
la valeur de bruit de fond est le mode des deuxièmes valeurs de mesure de signal obtenues à partir de la pluralité de sondes de correction.

14. Produit-programme informatique selon l'une quelconque des revendications 12 et 13, dans lequel
le microréseau a une pluralité de sondes de détection, et
les valeurs de correction sont obtenues en corrigeant, avec la valeur de bruit de fond, les premières valeurs de mesure de signal obtenues à partir de la pluralité de sondes de détection du microréseau.

15. Produit-programme informatique destiné à permettre à un ordinateur de détecter, avec un microréseau ayant une pluralité de sondes placées sur un substrat du microréseau, un acide nucléique cible issu d'un organisme contenant une séquence de reconnaissance qui est une séquence de bases à laquelle une protéine de liaison aux acides nucléiques est connue pour se lier spécifiquement, comprenant :
un support lisible par ordinateur ; et
des instructions logicielles, sur le support lisible par ordinateur, pour permettre à l'ordinateur d'effectuer des opérations prédéterminées comprenant :
(1) la réception de la séquence de reconnaissance et des séquences de base de la pluralité de sondes qui sont présentes dans un microréseau A mis en contact avec un échantillon d'analyte contenant potentiellement l'acide nucléique cible, et qui sont présentes dans un microréseau B mis en contact avec un échantillon témoin n'ayant aucune possibilité de contenir l'acide nucléique cible ;
(2) la sélection d'une sonde de détection parmi la pluralité de sondes présentes dans les microréseaux A et B, ladite sonde de détection ayant une séquence complémentaire de ladite séquence de reconnaissance ou une séquence complémentaire d'une région à moins de 300 bases de la séquence de reconnaissance dans la séquence d'acide nucléique cible, et étant susceptible de s'hybrider avec l'acide nucléique cible ;
(3) la sélection parmi la pluralité de sondes présentes dans les microréseaux A et B d'une sonde qui ne contient aucune séquence complémentaire de la séquence de reconnaissance et s'hybride avec un ADN génomique dudit organisme qui ne contient pas la séquence de reconnaissance dans la région à moins de 300 bases de la région d'hybridation à la sonde, en tant que sonde de correction ;
(4) l'obtention d'une première valeur de mesure de signal d'analyte dérivée d'une sonde de détection qui est présente dans un microréseau A, sélectionnée à l'étape (2) en contact avec un échantillon d'analyte contenant potentiellement l'acide nucléique cible, et
d'une deuxième valeur de mesure de signal d'analyte dérivée d'une sonde de correction qui est présente dans le microréseau A, sélectionnée à l'étape (3),
l'obtention d'une première valeur de mesure de signal de contrôle dérivée d'une sonde de détection présente dans un microréseau B, sélectionnée à l'étape (2) en contact avec un échantillon témoin, et
l'obtention d'une deuxième valeur de mesure de signal de contrôle dérivée d'une sonde de correction qui est présente dans le microréseau B, sélectionnée à l'étape (3) ;
(5) l'obtention d'une valeur de bruit de fond d'analyte et d'une valeur de bruit de fond de contrôle, respectivement, basées sur la deuxième valeur de mesure de signal d'analyte et la deuxième valeur de mesure de signal de contrôle ;
(6) l'obtention d'une valeur de correction d'analyte pour le premier signal d'analyte par correction de la première valeur de mesure de signal d'analyte avec la valeur de bruit de fond d'analyte, et
d'une valeur de correction de contrôle pour le premier signal de contrôle par correction de la première valeur de mesure de signal de contrôle avec la valeur de bruit de fond de contrôle ;
(7) l'obtention d'une valeur d'analyse basée sur la valeur de correction d'analyte et la valeur de correction de contrôle ; et
(8) la sortie de la valeur d'analyse.

16. Produit-programme informatique selon la revendication 15, dans lequel
les microréseaux A et B ont chacun une pluralité de sondes de correction,
la valeur de bruit de fond d'analyte est le mode des deuxièmes valeurs de mesure de signal d'analyte obtenues à partir de la pluralité de sondes de correction du microréseau A, et
la valeur de bruit de fond de contrôle est le mode des deuxièmes valeurs de mesure de signal de contrôle obtenues à partir de la pluralité de sondes de correction du microréseau B.

17. Produit-programme informatique selon l'une quelconque des revendications 15 et 16, dans lequel
les microréseaux A et B ont chacun une pluralité de sondes de détection,
les valeurs de correction d'analyte sont obtenues en soustrayant la valeur de bruit de fond d'analyte des premières valeurs de mesure de signal d'analyte obtenues à partir de la pluralité de sondes de détection du microréseau A,
les valeurs de correction de contrôle sont obtenues en soustrayant la valeur de bruit de fond de contrôle des premières valeurs de mesure de signal de contrôle obtenues à partir de la pluralité de sondes de détection du microréseau B, et
la valeur d'analyse est une probabilité de signification.


### SEQUENCE LISTING

<110> Sysmex Corporation
<120> METHODS OF DETECTING NUCLEIC ACID WITH MICROARRAY AND PROGRAM PRODUCT FOR
   USE IN MICROARRAY DATA ANALYSIS
<130> SYSM-036-EP
<150> JP 2009-155926
   <151> 2009-06-30
<150> JP 2010-068297
   <151> 2010-03-24
<160> 6
<170> PatentIn version 3.5
<210> 1
   <211> 18
   <212> DNA
   <213> Artificial Sequence
<220>
   <223> primer
<400> 1
   gaggccttcg ctggagtt 18
<210> 2
   <211> 20
   <212> DNA
   <213> Artificial Sequence
<220>
   <223> primer
<400> 2
   gtactcactg gtggcgaaga 20
<210> 3
   <211> 19
   <212> DNA
   <213> Artificial sequence
<220>
   <223> primer
<400> 3
   gcctacgagt tcaacgccg 19
<210> 4
   <211> 18
   <212> DNA
   <213> Artificial Sequence
<220>
   <223> primer
<400> 4
   aacgccgcag cctcagac 18
<210> 5
   <211> 25
   <212> DNA
   <213> Artificial Sequence
<220>
   <223> primer
<400> 5
   ggaggagtca agagaagttg gaagc 25
<210> 6
   <211> 23
   <212> DNA
   <213> Artificial Sequence
<220>
   <223> primer
<400> 6
   cccacactcc atttccattc ctc 23